# FASCICULE DE BREVET EUROPEEN

(11) **EP 3 394 019 B1**
(45) Date de publication et mention de la délivrance du brevet: **29.01.2020**
(21) Numéro de dépôt: 16809821.8
(22) Date de dépôt: 14.12.2016
(51) Int. Cl.: C07C 17/383, C07C 21/18

(54) **PROCÉDÉ DE PRODUCTION ET DE PURIFICATION DU 2,3,3,3-TÉTRAFLUOROPROPÈNE**
VERFAHREN ZUR HERSTELLUNG UND REINIGUNG VON 2,3,3,3-TETRAFLUORPROPEN
METHOD FOR PRODUCING AND PURIFYING 2,3,3,3-TETRAFLUOROPROPENE

(30) Priorité: 23.12.2015 FR 1563168
(43) Date de publication de la demande: 31.10.2018
(73) Titulaire: Arkema France, 92700 Colombes (FR)
(72) Inventeur: COLLIER, Bertrand, 69230 Saint-Genis-Laval (FR); DEUR-BERT, Dominique, 69390 Charly (FR); LACAMBRA, Joaquin, 64170 Serres Sainte Marie (FR); WENDLINGER, Laurent, 69510 Soucieu en Jarrest (FR)
(74) Mandataire: Leca, François Michel
(86) Numéro de dépôt international: PCT/EP2016/080949
(87) Numéro de publication internationale: WO 2017/108523

(56) Documents cités:
- WO-A1-2013/088195

## Description

### Domaine technique de l'invention

L'invention se rapporte à un procédé de purification du 2,3,3,3-tetrafluoro-1-propène. En outre, l'invention se rapporte également à un procédé de production et de purification du 2,3,3,3-tetrafluoro-1-propène.

### Arrière-plan technologique de l'invention

Les hydrofluorocarbures (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. Les HFO ont été identifiés comme des alternatives souhaitables au HCFC du fait de leurs faibles valeurs d'ODP (Ozone Depletion Potential ou potentiel d'appauvrissement de la couche d'ozone) et de GWP (Global Warming Potential ou potentiel de réchauffement climatique).

La plupart des procédés de fabrication des hydrofluorooléfines font appel à une réaction de fluoration et/ou de dehydrohalogénation. Ce type de réaction est effectué en phase gazeuse et génère des impuretés qu'il faut par conséquent éliminer pour obtenir le composé désiré dans un degré de pureté suffisant pour les applications visées.

Par exemple, dans le cadre de la production de 2,3,3,3-tétrafluoro-1-propène (HFO-1234yf), la présence d'impuretés telles que le 1-chloro-3,3,3-trifluoro-1-propene (1233zd), 1,3,3,3-tetrafluoro-1-propene (1234ze) et le 1,1,1,3,3-pentafluoropropane (245fa) sont observées. Ces impuretés sont des isomères des composés principaux visant à être obtenues par le procédé de production du 2,3,3,3-tétrafluoro-1-propène outre ce dernier, i.e. 2-chloro-3,3,3-trifluoro-1-propene (1233xf) et 1,1,1,2,2-pentafluoropropane (245cb). Compte tenu des points d'ébullition respectifs du 1-chloro-3,3,3-trifluoro-1-propene (1233zd), 1,3,3,3-tetrafluoro-1-propene (1234ze) et le 1,1,1,3,3-pentafluoropropane (245fa), ceux-ci peuvent s'accumuler dans la boucle réactionnelle et ainsi empêcher la formation des produits d'intérêt.

La purification de ce type de mélange réactionnel peut être effectuée par différentes techniques connues de l'art antérieur, telles que par exemple la distillation. Cependant lorsque les composés à purifier ont des points d'ébullition trop proches ou lorsque ceux-ci forment des compositions azéotropes ou quasi-azéotropes, la distillation n'est pas un procédé efficace. Des procédés de distillation extractive ont ainsi été décrits.

On connaît par EP 0 864 554 un procédé de purification d'un mélange comprenant 1,1,1,3,3-pentafluoropropane (245fa) et 1-chloro-3,3,3-trifluoro-trans-1-propene (1233zd) par distillation en présence d'un solvant ayant un point d'ébullition supérieur à celui de 1-chloro-3,3,3-trifluoro-trans-1-propene.

On connaît par WO 03/068716 un procédé de récupération de pentafluoroéthane à partir d'un mélange comprenant du pentafluoroéthane et du chloropentafluoroéthane par distillation en présence d'hexafluoropropène.

On connaît aussi par WO 98/19982 un procédé de purification du 1,1-difluoroéthane par distillation extractive. Le procédé consiste à mettre en contact un agent d'extraction avec un mélange de 1,1-difluoroéthane et de chlorure de vinyle. L'agent d'extraction est choisi parmi les hydrocarbures, les alcools, les chlorocarbures ayant un point d'ébullition compris entre 10°C et 120°C. Comme mentionné par WO 98/19982, la sélection de l'agent d'extraction peut s'avérer complexe en fonction des produits à séparer. On connaît par WO 2013/088195 un procédé de préparation de 2,3,3,3-tetrafluoropropene à partir de 1,1,1,2,3-pentachloropropane et/ou 1,1,2,2,3-pentachloropropane. Il existe donc toujours un besoin pour la mise en œuvre d'un procédé particulier de purification du 2,3,3,3-tetrafluoro-1-propène.

### Résumé de l'invention

Dans un procédé de production du 2,3,3,3-tetrafluoro-1-propène, le choix de conditions opérationnelles particulières peut favoriser la présence de certaines impuretés ou d'isomères de celles-ci. La présence d'impuretés telles que 1,3,3,3-tetrafluoro-1-propene (1234ze) peut être observée tout comme celle de 1-chloro-3,3,3-trifluoro-1-propene (1233zd), et 1,1,1,3,3-pentafluoropropane (245fa). Ces impuretés peuvent provenir de réactions secondaires induites par des composés produits intermédiairement pendant la production du 2,3,3,3-tetrafluoro-1-propène, et peuvent présenter des propriétés physiques telles que leur élimination peut s'avérer complexe. La présente invention permet notamment la production de 2,3,3,3-tetrafluoro-1-propène avec une pureté améliorée.

Selon un premier aspect, l'invention fournit un procédé de production et de purification du 2,3,3,3-tétrafluoropropène (1234yf) mis en œuvre à partir d'une composition de départ comprenant au moins un composé de formule CX(Y)₂-CX(Y)ₘ-CHₘXY (I) dans laquelle X et Y représentent indépendamment H, F, ou Cl et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;
ledit procédé comprenant les étapes de :
a) mise en contact, en présence d'un catalyseur, de la composition de départ avec HF pour produire une composition **A** comprenant HCl, une partie du HF n'ayant pas réagi, 2,3,3,3-tétrafluoropropène (1234yf), des produits intermédiaires **B** consistant en 2-chloro-3,3,3-trifluoropropène (1233xf), 1,1,1,2,2-pentafluoropropane (245cb), et des produits secondaires **C** consistant en E-1-chloro-3,3,3-trifluoro-1-propene (1233zdE), trans-1,3,3,3-tetrafluoro-1-propene (1234zeE) et 1,1,1,3,3-pentafluoropropane (245fa);
b) récupération de ladite composition **A** et purification, de préférence distillation, de celle-ci pour former et récupérer un premier courant, de préférence gazeux, comprenant HCl, 2,3,3,3-tétrafluoropropène (1234yf), une partie des produits intermédiaires **B** et une partie des produits secondaires **C**; et un courant, de préférence liquide, **L1** comprenant une partie du HF n'ayant pas réagi, une partie des produits intermédiaires **B** et une partie des produits secondaires **C** ;
c) purification dudit premier courant pour former un courant comprenant une portion de ladite partie des produits intermédiaires **B** et une partie des produits secondaires **C** et recyclage de celui-ci à l'étape a).

Selon un mode de réalisation préféré, ledit premier courant est un courant gazeux **G1** purifié par les étapes suivantes :
b1) distillation du courant gazeux **G1** pour récupérer un courant **G1a** comprenant du HCl, avantageusement en tête de colonne de distillation, et un courant **G1b** comprenant 2,3,3,3-tétrafluoropropène (1234yf), ladite une partie des produits intermédiaires **B** et ladite une partie des produits secondaires **C,** avantageusement en bas de colonne de distillation ;
b2) distillation dudit courant **G1b** obtenu à l'étape b1) pour former un courant **G1c** comprenant 2,3,3,3-tétrafluoropropène (1234yf), une portion de ladite partie des produits intermédiaires **B** et une portion de ladite partie des produits secondaires **C,** avantageusement en tête de colonne de distillation, et un courant **G1d** comprenant une portion de ladite partie des produits intermédiaires **B** et une portion de ladite partie des produits secondaires **C,** avantageusement en bas de colonne de distillation, de préférence le courant **G1d** est recyclé à l'étape a).

De préférence, le courant **G1c** formé à l'étape b2) peut comprendre 2,3,3,3-tétrafluoropropène (1234yf), 1,1,1,2,2-pentafluoropropane (245cb) et trans-1,3,3,3-tetrafluoro-1-propene (1234zeE). De préférence, le courant **G1d** formé à l'étape b2) peut comprendre 1,1,1,2,2-pentafluoropropane (245cb), trans-1,3,3,3-tetrafluoro-1-propene (1234zeE), 2-chloro-3,3,3-trifluoropropène (1233xf). En particulier, la teneur en 1,1,1,2,2-pentafluoropropane (245cb) est plus importante dans le courant **G1d** que dans le courant **G1c.**

Selon un mode de réalisation préféré, le procédé comprend une étape b3), subséquente à l'étape b2), dans laquelle le courant **G1c** obtenu à l'étape b2) comprend 2,3,3,3-tétrafluoropropène (1234yf), 1,1,1,2,2-pentafluoropropane (245cb) et trans-1,3,3,3-tetrafluoro-1-propene (1234zeE) ; et ledit courant **G1c** est distillé pour former un courant **G1e** comprenant 2,3,3,3-tétrafluoropropène (1234yf) et un courant **G1f** comprenant 1,1,1,2,2-pentafluoropropane (245cb) et trans-1,3,3,3-tetrafluoro-1-propene (1234zeE). De préférence, le courant **G1e** comprenant 2,3,3,3-tétrafluoropropène (1234yf) peut être soumis à des étapes de purification ultérieures afin d'obtenir un degré de pureté suffisant pour sa commercialisation.

Selon un mode de réalisation préféré, le courant **G1f** obtenu à l'étape b3) est séparé par distillation extractive.

Selon un mode de réalisation particulier, le courant **G1f** obtenu à l'étape b3) est séparé par distillation extractive suivant les étapes :
b4) mise en contact dudit courant **G1f** obtenu à l'étape b3) avec un agent d'extraction organique pour former un courant **G1g,** et
b5) distillation extractive du courant **G1g** pour former un flux **G1h** comprenant 1,1,1,2,2-pentafluoropropane (245cb), avantageusement en tête de colonne de distillation, et un courant **G1i** comprenant trans-1,3,3,3-tetrafluoro-1-propene (1234zeE) et ledit agent d'extraction organique, avantageusement en bas de colonne de distillation.

De préférence, le courant **G1i** comprenant trans-1,3,3,3-tetrafluoro-1-propene (1234zeE) et ledit agent d'extraction organique est séparé par distillation pour former un courant **G1j** comprenant ledit agent d'extraction organique et un courant **G1k** comprenant trans-1,3,3,3-tetrafluoro-1-propene (1234zeE). Le courant **G1j** comprenant ledit agent d'extraction organique peut être recyclé à l'étape b4). Le courant **G1k** comprenant trans-1,3,3,3-tetrafluoro-1-propene (1234zeE) peut être soit purifié ou détruit par incinération.

De préférence, le flux **G1h** comprenant 1,1,1,2,2-pentafluoropropane (245cb), de préférence dépourvu de trans-1,3,3,3-tetrafluoro-1-propene (1234zeE), est recyclé à l'étape a).

Alternativement, le procédé comprend une étape b3'), subséquente à l'étape b2), dans laquelle le courant **G1c** obtenu à l'étape b2) comprend 2,3,3,3-tétrafluoropropène (1234yf), 1,1,1,2,2-pentafluoropropane (245cb) et trans-1,3,3,3-tetrafluoro-1-propene (1234zeE) ; et ledit courant **G1c** est distillé pour former un courant **G1e'** comprenant 2,3,3,3-tétrafluoropropène (1234yf) et 1,1,1,2,2-pentafluoropropane (245cb) et un courant **G1f'** comprenant trans-1,3,3,3-tetrafluoro-1-propene (1234zeE). Ledit courant **G1c** est distillé par distillation extractive pour former ledit courant **G1e'** comprenant 2,3,3,3-tétrafluoropropène (1234yf) et 1,1,1,2,2-pentafluoropropane (245cb) ; et ledit courant **G1f'** comprenant trans-1,3,3,3-tetrafluoro-1-propene (1234zeE). Ainsi, le courant **G1c** est distillé par distillation extractive suivant les étapes :
b4') mise en contact dudit courant **G1c** obtenu à l'étape b2) avec un agent d'extraction organique pour former un courant **G1g',** et
b5') distillation extractive du courant **G1g'** pour former un flux **G1e'** comprenant 2,3,3,3-tetrafluoropropene (1234yf) et 1,1,1,2,2-pentafluoropropane (245cb), avantageusement en tête de colonne de distillation, et un courant **G1h'** comprenant trans-1,3,3,3-tetrafluoro-1-propene (1234zeE) et ledit agent d'extraction organique, avantageusement en bas de colonne de distillation.

De préférence, le courant **G1e'** comprenant 2,3,3,3-tétrafluoropropène (1234yf) et 1,1,1,2,2-pentafluoropropane (245cb) peut être soumis à des étapes de purification ultérieures. Ainsi, le 2,3,3,3-tétrafluoropropène (1234yf) peut être séparé, de préférence par distillation du 1,1,1,2,2-pentafluoropropane (245cb) pour former un courant comprenant 2,3,3,3-tétrafluoropropène (1234yf) et un courant **G1i'** comprenant 1,1,1,2,2-pentafluoropropane (245cb), ledit courant **G1i'** étant recyclé à l'étape a). Le 2,3,3,3-tétrafluoropropène (1234yf) peut encore être soumis à des étapes de purification ultérieures afin d'obtenir un degré de pureté suffisant pour sa commercialisation.

De préférence, le courant **G1h'** comprenant trans-1,3,3,3-tetrafluoro-1-propene (1234zeE) et ledit agent d'extraction organique est séparé par distillation pour former un courant **G1j'** comprenant ledit agent d'extraction organique et un courant **G1k'** comprenant trans-1,3,3,3-tetrafluoro-1-propene (1234zeE). Le courant **G1j'** comprenant ledit agent d'extraction organique peut être recyclé à l'étape b4'). Le courant **G1k'** comprenant trans-1,3,3,3-tetrafluoro-1-propene (1234zeE) peut être soit purifié ou détruit par incinération.

Selon un autre mode de réalisation préféré, ledit courant liquide **L1** comprend, outre le HF n'ayant pas réagi, tout ou partie des produits intermédiaires **B** et tout ou partie des produits secondaires **C** ; et tout ou partie de ce courant **L1** est porté à basse température, avantageusement entre -50°C et 15°C, de préférence entre -30°C à 0°C, pour former une première phase **L1a** comprenant une partie du HF n'ayant pas réagi et une seconde phase **L1b** comprenant lesdits produits intermédiaires **B** et lesdits produits secondaires **C**; optionnellement ou non, ledit courant **G1d** formé à l'étape b2) est mélangé au courant liquide **L1** avant que ce dernier soit porté à basse température.

De préférence, ledit courant liquide **L1** comprend une partie des produits intermédiaires **B** et tout ou partie des produits secondaires **C,** et une partie du courant liquide **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C** ; optionnellement ou non, ledit courant **G1d** formé à l'étape b2) est mélangé au courant liquide **L1** avant que ce dernier soit porté à basse température.

De préférence, ladite première phase **L1a** est recyclée à l'étape a).

Selon un mode de réalisation préféré, ladite seconde phase **L1b** est distillée pour récupérer un courant **L1c** comprenant 1,1,1,2,2-pentafluoropropane (245cb) et trans-1,3,3,3-tetrafluoro-1-propene (1234zeE), avantageusement en tête de colonne de distillation, et un courant **L1d** comprenant 2-chloro-3,3,3-trifluoro-1-propene (1233xf), E-1-chloro-3,3,3-trifluoro-1-propene (1233zdE) et 1,1,1,3,3-pentafluoropropane (245fa) ; avantageusement en bas de colonne de distillation, avantageusement ledit courant **L1c** est recyclé à l'étape a).

Selon un mode de réalisation préféré, ledit courant **L1d** est séparé pour former un flux comprenant 2-chloro-3,3,3-trifluoro-1-propene (1233xf) et un courant comprenant E-1-chloro-3,3,3-trifluoro-1-propene (1233zdE) et 1,1,1,3,3-pentafluoropropane (245fa).

Selon un mode de réalisation préféré, la séparation dudit courant **L1d** est effectuée par distillation extractive.

Selon un mode de réalisation particulier, la distillation extractive dudit courant **L1d** comprend les étapes de :
- mise en contact dudit courant **L1d** avec un agent d'extraction organique pour former une composition **L1e**, et
- distillation extractive de la composition **L1e** pour former un flux **L1f** comprenant 2-chloro-3,3,3-trifluoro-1-propene (1233xf), avantageusement en tête de colonne de distillation, et un courant **L1g** comprenant E-1-chloro-3,3,3-trifluoro-1-propene (1233zdE) et 1,1,1,3,3-pentafluoropropane (245fa) et ledit agent d'extraction organique, avantageusement en bas de colonne de distillation.

De préférence, le courant **L1g** est ensuite séparé par distillation pour former un courant **L1h** comprenant ledit agent d'extraction organique et un courant **L1i** comprenant E-1-chloro-3,3,3-trifluoro-1-propene (1233zdE) et 1,1,1,3,3-pentafluoropropane (245fa). Le courant **L1h** peut être recyclé pour être mis en contact avec un courant **L1d** pour former une composition **L1e.** Le courant **L1i** comprenant E-1-chloro-3,3,3-trifluoro-1-propene (1233zdE) et 1,1,1,3,3-pentafluoropropane (245fa) peut être soit purifié soit détruit par incinération.

De préférence, le flux **L1f** comprenant 2-chloro-3,3,3-trifluoro-1-propene (1233xf), de préférence dépourvu de 1,1,1,3,3-pentafluoropropane (245fa) et de E-1-chloro-3,3,3-trifluoro-1-propene (1233zdE) est recyclé à l'étape a).

Selon un mode de réalisation préféré, la présente invention permet ainsi le recyclage à l'étape a) d'un ou plusieurs flux dépourvus d'un ou plusieurs produits secondaires **C.**

### Brève description des dessins

La figure 1 représente schématiquement un dispositif mettant en œuvre un procédé de production du 2,3,3,3-tetrafluoro-1-propène selon un mode de réalisation particulier de la présente invention.
Les figures 2 et 3 représentent schématiquement un dispositif mettant en œuvre la purification du 2-3,3,3-tetrafluoro-1-propène selon un mode de réalisation particulier de la présente invention.

### Description détaillée de l'invention

La présente invention permet la production et la purification du 2,3,3,3-tétrafluoropropène (1234yf). Selon un premier aspect de la présente invention, un procédé de production et de purification du 2,3,3,3-tétrafluoropropène (1234yf) est fourni. Ledit procédé est mis en œuvre à partir d'une composition de départ comprenant au moins un composé de formule CX(Y)₂-CX(Y)ₘ-CHₘXY (I) dans laquelle X et Y représentent indépendamment H, F, ou Cl et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1.

De préférence, ledit procédé comprend les étapes de :
a) mise en contact, en présence d'un catalyseur, de la composition de départ avec HF pour produire une composition **A** comprenant 2,3,3,3-tétrafluoropropène (1234yf), des produits intermédiaires **B** consistant en 2-chloro-3,3,3-trifluoropropène (1233xf), 1,1,1,2,2-pentafluoropropane (245cb), et des produits secondaires **C** consistant en 1-chloro-3,3,3-trifluoro-1-propene (1233zd), 1,3,3,3-tetrafluoro-1-propene (1234ze) et 1,1,1,3,3-pentafluoropropane (245fa) ;
b) récupération de ladite composition **A** et purification de celle-ci pour former et récupérer un premier courant comprenant 2,3,3,3-tétrafluoropropène (1234yf) et un ou plusieurs flux comprenant 2-chloro-3,3,3-trifluoropropène (1233xf) et/ou 1,1,1,2,2-pentafluoropropane (245cb) ;
c) recyclage à l'étape a) desdits un ou plusieurs flux comprenant 2-chloro-3,3,3-trifluoropropène (1233xf) et/ou 1,1,1,2,2-pentafluoropropane (245cb).

De préférence, la teneur d'au moins un des produits secondaires **C** dans lesdits un ou plusieurs flux recyclé à l'étape a) peut être inférieure à celle-ci dans ladite composition **A.**

La teneur en l'un quelconque des produits secondaires **C** peut être diminuée dans un ou plusieurs ou tout desdits un ou plusieurs flux recyclés à l'étape a). De préférence, ladite teneur en au moins un des produits secondaires **C** dans lesdits un ou plusieurs flux recyclés à l'étape a) peut être diminuée de 20%, 30%, 40%, 50%, 60%, 70%, 80%, 90%, 95% ou 98% par rapport à ladite teneur du même au moins un des produits secondaires **C** dans ladite composition **A.** Ainsi, la teneur en E-1-chloro-3,3,3-trifluoro-1-propene (1233zdE) dans lesdits un ou plusieurs flux recyclé à l'étape a) peut être diminuée de 20%, 30%, 40%, 50%, 60%, 70%, 80%, 90%, 95% ou 98% par rapport à la teneur en E-1-chloro-3,3,3-trifluoro-1-propene (1233zdE) dans ladite composition **A.** Selon un autre mode de réalisation, la teneur en trans-1,3,3,3-tetrafluoro-1-propene (1234zeE) dans lesdits un ou plusieurs flux recyclé à l'étape a) peut être diminuée de 20%, 30%, 40%, 50%, 60%, 70%, 80%, 90%, 95% ou 98% par rapport à la teneur en trans-1,3,3,3-tetrafluoro-1-propene (1234zeE) dans ladite composition **A.** Selon un autre mode de réalisation, la teneur en 1,1,1,3,3-pentafluoropropane (245fa) dans lesdits un ou plusieurs flux recyclé à l'étape a) peut être diminuée de 20%, 30%, 40%, 50%, 60%, 70%, 80%, 90%, 95% ou 98% par rapport à la teneur en 1,1,1,3,3-pentafluoropropane (245fa) dans ladite composition **A.** Les teneurs sont exprimées en poids.

Lesdits un ou plusieurs flux recyclés à l'étape a) peuvent être dépourvus d'un ou plusieurs des produits secondaires **C.** Le terme « dépourvu » signifie que le courant considéré comprenant moins de 50 ppm, avantageusement moins de 20 ppm, de préférence moins de 10 ppm du composé considéré sur base du poids total dudit courant.

Selon un mode de réalisation préféré, la composition **A** comprend également HCl, une partie du HF. De préférence, la purification de ladite composition **A** effectuée à l'étape b) comprend la distillation de ladite composition **A** pour récupérer en tête de colonne de distillation un courant gazeux **G1** comprenant HCl et 2,3,3,3-tétrafluoropropène (1234yf); et en bas de colonne de distillation un courant liquide **L1** comprenant ladite une partie du HF. Tout ou partie des produits intermédiaires **B** et tout ou partie des produits secondaires C peuvent être contenus dans ledit courant gazeux **G1** et/ou dans ledit courant liquide **L1.**

De préférence, tout ou partie du 1,1,1,2,2-pentafluoropropane (245cb) peut être contenu dans ledit courant gazeux **G1**. Tout ou partie du 1,1,1,2,2-pentafluoropropane (245cb) peut aussi être contenu dans ledit courant liquide **L1.**

De préférence, tout ou partie du trans-1,3,3,3-tetrafluoro-1-propene (1234zeE) peut être contenu dans ledit courant gazeux **G1**. Tout ou partie du trans-1,3,3,3-tetrafluoro-1-propene (1234zeE) peut aussi être contenu dans ledit courant liquide **L1.**

De préférence, tout ou partie du 2-chloro-3,3,3-trifluoropropène (1233xf) peut être contenu dans ledit courant gazeux **G1**. Tout ou partie du 2-chloro-3,3,3-trifluoropropène (1233xf) peut aussi être contenu dans ledit courant liquide **L1.** De manière privilégiée, le 2-chloro-3,3,3-trifluoropropène (1233xf) est contenu dans ledit courant liquide **L1**, avantageusement 70%, 75%, 80%, 85% ou 90% du 2-chloro-3,3,3-trifluoropropène (1233xf) est contenu dans ledit courant liquide **L1** par rapport audit courant gazeux **G1**.

De préférence, tout ou partie du E-1-chloro-3,3,3-trifluoro-1-propene (1233zdE) peut être contenu dans ledit courant gazeux **G1**. Tout ou partie du E-1-chloro-3,3,3-trifluoro-1-propene (1233zdE) peut aussi être contenu dans ledit courant liquide **L1.** De manière privilégiée, le E-1-chloro-3,3,3-trifluoro-1-propene (1233zdE) est contenu dans ledit courant liquide **L1,** avantageusement 70%, 75%, 80%, 85%, 90% ou 95% du 1-chloro-3,3,3-trifluoro-1-propene (1233zd) est contenu dans ledit courant liquide **L1** par rapport audit courant gazeux **G1**.

De préférence, tout ou partie du 1,1,1,3,3-pentafluoropropane (245fa) peut être contenu dans ledit courant gazeux **G1**. Tout ou partie du 1,1,1,3,3-pentafluoropropane (245fa) peut aussi être contenu dans ledit courant liquide **L1.** De manière privilégiée, le 1,1,1,3,3-pentafluoropropane (245fa) est contenu dans ledit courant liquide **L1**, avantageusement 70%, 75%, 80%, 85%, 90% ou 95% du 1,1,1,3,3-pentafluoropropane (245fa) est contenu dans ledit courant liquide **L1** par rapport audit courant gazeux **G1**.

Selon un mode de réalisation préféré, ledit courant gazeux **G1** comprend une partie des produits intermédiaires **B** et une partie des produits secondaires **C,** et ledit courant gazeux **G1** est purifié par les étapes suivantes :
b1) distillation du courant gazeux **G1** pour récupérer un courant **G1a** comprenant du HCl, avantageusement en tête de colonne de distillation, et un courant **G1b** comprenant 2,3,3,3-tétrafluoropropène (1234yf), ladite une partie des produits intermédiaires **B** et ladite une partie des produits secondaires **C,** avantageusement en bas de colonne de distillation ;
b2) distillation dudit courant **G1b** obtenu à l'étape b1) pour former un courant **G1c** comprenant 2,3,3,3-tétrafluoropropène (1234yf), une portion de ladite partie des produits intermédiaires **B** et une portion de ladite partie des produits secondaires **C,** avantageusement en tête de colonne de distillation, et un courant **G1d** comprenant une portion de ladite partie des produits intermédiaires **B** et une portion de ladite partie des produits secondaires **C,** avantageusement en bas de colonne de distillation.

De préférence, le courant **G1c** formé à l'étape b2) peut comprendre 2,3,3,3-tétrafluoropropène (1234yf), 1,1,1,2,2-pentafluoropropane (245cb) et 1,3,3,3-tetrafluoro-1-propene (1234ze). De préférence, le courant **G1d** formé à l'étape b2) peut comprendre 1,1,1,2,2-pentafluoropropane (245cb), trans-1,3,3,3-tetrafluoro-1-propene (1234zeE), 2-chloro-3,3,3-trifluoropropène (1233xf) et optionnellement ou non E-1-chloro-3,3,3-trifluoro-1-propene (1233zdE) et 1,1,1,3,3-pentafluoropropane (245fa). En particulier, la teneur en 1,1,1,2,2-pentafluoropropane (245cb) est plus importante dans le courant **G1d** que dans le courant **G1c.** Le courant **G1d** peut contenir 55%, 60%, 65%, 70%, 75%, 78% ou 80% du 1,1,1,2,2-pentafluoropropane (245cb) sur base de la teneur totale en 1,1,1,2,2-pentafluoropropane (245cb) dans le courant **G1d** et **G1c.**

Le courant **G1d** peut être recyclé à l'étape a) du présent procédé. Le courant **G1d** peut être un desdits un ou plusieurs flux recyclé à l'étape a) (étape c) du présent procédé.

Ainsi, le présent procédé peut être un procédé de production et de purification du 2,3,3,3-tétrafluoropropène (1234yf) mis en œuvre à partir d'une composition de départ comprenant au moins un composé de formule (I) CX(Y)₂-CX(Y)ₘ-CHₘXY dans laquelle X et Y représentent indépendamment un atome d'hydrogène, de fluor ou de chlore et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ; ledit procédé comprenant les étapes de :
a) mise en contact, en présence d'un catalyseur, de la composition de départ avec HF pour produire une composition **A** comprenant HCl, une partie du HF n'ayant pas réagi, 2,3,3,3-tétrafluoropropène (1234yf), des produits intermédiaires **B** consistant en 2-chloro-3,3,3-trifluoropropène (1233xf), 1,1,1,2,2-pentafluoropropane (245cb), et des produits secondaires **C** consistant en E-1-chloro-3,3,3-trifluoro-1-propene (1233zdE), trans-1,3,3,3-tetrafluoro-1-propene (1234zeE) et 1,1,1,3,3-pentafluoropropane (245fa);
b) récupération de ladite composition **A** et distillation de celle-ci pour former et récupérer en tête de colonne de distillation un courant gazeux **G1** comprenant HCl et 2,3,3,3-tétrafluoropropène (1234yf), une partie des produits intermédiaires **B** et une partie des produits secondaires **C;** et en bas de colonne de distillation un courant liquide **L1** comprenant ladite une partie du HF n'ayant pas réagi, une partie des produits intermédiaires **B** et une partie des produits secondaires **C** ; ledit courant gazeux **G1** étant purifié par les étapes suivantes :
   b1) distillation du courant gazeux **G1** pour récupérer un courant **G1a** comprenant du HCl et un courant **G1b** comprenant 2,3,3,3-tétrafluoropropène (1234yf), ladite une partie des produits intermédiaires **B** et ladite une partie des produits secondaires **C** ;
   b2) distillation dudit courant **G1b** obtenu à l'étape b1) pour former un courant **G1c** comprenant 2,3,3,3-tétrafluoropropène (1234yf), une portion de ladite partie des produits intermédiaires **B** et une portion de ladite partie des produits secondaires **C** et un courant **G1d** comprenant une portion de ladite partie des produits intermédiaires **B** et une portion de ladite partie des produits secondaires **C,**
c) recyclage à l'étape a) du courant **G1d,**
   de préférence, la teneur d'au moins un des produits secondaires C dans le courant **G1d** étant inférieure à celle-ci dans ladite composition **A.**

Selon un mode de réalisation préféré, le procédé comprend une étape b3), subséquente à l'étape b2), dans laquelle le courant **G1c** obtenu à l'étape b2) comprend 2,3,3,3-tétrafluoropropène (1234yf), 1,1,1,2,2-pentafluoropropane (245cb) et trans-1,3,3,3-tetrafluoro-1-propene (1234zeE) ; et ledit courant **G1c** est distillé pour former un courant **G1e** comprenant 2,3,3,3-tétrafluoropropène (1234yf) et un courant **G1f** comprenant 1,1,1,2,2-pentafluoropropane (245cb) et trans-1,3,3,3-tetrafluoro-1-propene (1234zeE).

Le courant **G1f** obtenu à l'étape b3) peut être séparé par distillation extractive.

Selon un mode de réalisation particulier, le courant **G1f** obtenu à l'étape b3) est séparé par distillation extractive suivant les étapes :
b4) mise en contact dudit courant **G1f** obtenu à l'étape b3) avec un agent d'extraction organique pour former un courant **G1g,** et
b5) distillation extractive du courant **G1g** pour former un flux **G1h** comprenant 1,1,1,2,2-pentafluoropropane (245cb), avantageusement en tête de colonne de distillation, et une composition **G1i** comprenant trans-1,3,3,3-tetrafluoro-1-propene (1234zeE) et ledit agent d'extraction organique, avantageusement en bas de colonne de distillation.

De préférence, le courant **G1i** comprenant trans-1,3,3,3-tetrafluoro-1-propene (1234zeE) et ledit agent d'extraction organique est séparé par distillation pour former un courant **G1j** comprenant ledit agent d'extraction organique et un courant **G1k** comprenant trans-1,3,3,3-tetrafluoro-1-propene (1234zeE). Le courant **G1j** comprenant ledit agent d'extraction organique peut être recyclé à l'étape b4). Le courant **G1k** comprenant trans-1,3,3,3-tetrafluoro-1-propene (1234zeE) peut être soit purifié ou détruit par incinération.

Selon un mode de réalisation préféré, ledit agent d'extraction organique est un solvant choisi parmi le groupe consistant en hydrohalocarbure, alcool, cétone, amine, ester, éther, aldéhyde, nitrile, carbonate, thioalkyle, amide, hétérocycle. Avantageusement, ledit agent d'extraction organique est un solvant sélectionné parmi le groupe consistant en alcool, cétone, amine, ester et hétérocycle. Selon un mode de réalisation préféré, ledit agent d'extraction organique a un point d'ébullition compris entre 10 et 150°C.

De préférence, ledit agent d'extraction peut avoir un facteur de séparation S_{1,2} supérieur ou égal à 1,1, ledit facteur de séparation étant calculé par la formule S_{1,2} = (γ_{1,S}*P1)/(γ_{2,S}*P2) dans laquelle
γ_{1,S} représente le coefficient d'activité du 1,1,1,2,2-pentafluoropropane dans ledit agent d'extraction organique à dilution infinie,
P1 représente la pression de vapeur saturante du 1,1,1,2,2-pentafluoropropane,
γ_{2,S} représente le coefficient d'activité dudit trans-1,3,3,3-tetrafluoro-1-propene (1234zeE) dans ledit agent d'extraction organique à dilution infinie,
P2 représente la pression de vapeur saturante dudit trans-1,3,3,3-tetrafluoro-1-propene (1234zeE) ;
avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

La pression de vapeur saturante est considérée pour une température de 25°C.

De préférence, ledit agent d'extraction organique peut avoir une capacité de séparation C_{2,S} supérieure ou égale à 0,20, ladite capacité de séparation étant calculée par la formule C_{2,S} = 1/(γ_{2,S}) dans laquelle γ_{2,S} représente le coefficient d'activité dudit trans-1,3,3,3-tetrafluoro-1-propene (1234zeE) dans ledit agent d'extraction organique à dilution infinie ; avantageusement, la capacité de séparation C_{2,S} est supérieure ou égale à 0,40, de préférence supérieure ou égale à 0,60, plus préférentiellement supérieure ou égale à 0,80, en particulier supérieure ou égale à 1,0.

De préférence, ledit agent d'extraction organique peut avoir un facteur de séparation S_{1,2} supérieur ou égal à 1,5 et une capacité d'absorption C_{2,S} supérieure ou égale à 0,6 et être sélectionné parmi le groupe consistant en éthylamine, acetaldehyde, isopropylamine, methylformate, diethylether, 1,2-epoxypropane, ethylmethylamine, dimethoxymethane, 2-amino-2-methylpropane, methylcyclopropylether, n-propylamine, isopropylmethylamine, 2-ethoxy-propane, methyl-t-butylether, diethylamine, propanone, methylacetate, 4-methoxy-2-methyl-2-butanethiol, 2-butanamine, n-methylpropylamine, isobutanal, tetrahydrofurane, isopropylformate, diisopropylether, 2-ethoxy-2-methyl-propane, 1-butylamine, ethylacetate, butanone, n-propylformate, 2-ethoxy-butane, 1-methoxy-2-methyl-butane, 2,2-dimethoxypropane, 1-ethoxy-2-methylpropane, diisopropylamine, 1,2-dimethoxyethane, 3-methyl-2-butanamine, diethoxymethane, isopropylacetate, di-n-propylether, 3-pentylamine, n-methylbutylamine, 1-ethoxybutane, 1-methoxy-2-propanamine, 2-methylbutanal, 2-methoxyethanamine, tert-butylacetate, propionitrile, 2-allyloxyethanol, 1-methoxy-pentane, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, dipropylamine, 2-ethoxyethanamine, sec-butylacetate, n-methyl-1,2-ethanediamine, 2,2-diethoxypropane, pyridine, 4-methyl-2-pentanone, 1,2-diaminoethane, butyronitrile, sec-butyl-tert-butylether, 1-methoxy2-propanol, 1,2-propanediamine, 2,6-dimethyl-5-heptenal, 1-(dimethylamino)-2-propanol, 3-methyl-3-pentanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-methylpyrazine, 2-ethoxy-1-propanol, 1-methylpiperazine, 1,3-propanediamine, di-n-butylether, valeronitrile, 2-heptanamine, 1-ethoxy-hexane, n,n-diethylethylenediamine, 2,6-dimethylpyridine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol. Avantageusement, ledit agent d'extraction organique peut avoir un facteur de séparation S_{1,2} supérieur ou égal à 1,8 et/ou une capacité d'absorption C_{2,S} supérieure ou égale à 0,8 ; et être sélectionné parmi le groupe consistant en éthylamine, isopropylamine, ethylmethylamine, 2-amino-2-methylpropane, n-propylamine, isopropylmethylamine, diethylamine, propanone, 2-butanamine, n-methylpropylamine, tetrahydrofurane, 1-butylamine, ethylacetate, butanone, 1,2-dimethoxyethane, 3-methyl-2-butanamine, 3-pentylamine, n-methylbutylamine, 1-methoxy-2-propanamine, 2-methoxyethanamine, ethylpropionate, dioxane, 3-pentanone, 2-pentanone, 2-methoxy-1propanamine, trimethoxymethane, n-pentylamine, 3,3-dimethyl-2-butanone, 1,3-dioxane, piperidine, 2-ethoxyethanamine, n-methyl-1,2-ethanediamine, 1,2-diaminoethane, butyronitrile, 1-methoxy2-propanol, 1,2-propanediamine, 1-(dimethylamino)-2-propanol, 2-ethylbutylamine, diethylcarbonate, n-butylacetate, 2-hexanone, n-ethylethylenediamine, 2-methoxy1-propanol, 1, ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, 2-ethoxy-1-propanol, 1-methylpiperazine, 1,3-propanediamine, valeronitrile, 2-heptanamine, n,n-diethylethylenediamine, 4-methyl-2-hexanone, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol. De préférence, ledit agent d'extraction organique peut avoir un facteur de séparation S_{1,2} supérieur ou égal à 1,9 et/ou une capacité d'absorption C_{2,S} supérieure ou égale à 0,9 et être sélectionné parmi le groupe consistant en éthylamine, isopropylamine, ethylmethylamine, 2-amino-2-methylpropane, n-propylamine, isopropylmethylamine, diethylamine, propanone, 2-butanamine, n-methylpropylamine, tetrahydrofurane, 1-butylamine, ethylacetate, butanone, 1,2-dimethoxyethane, 3-methyl-2-butanamine, 3-pentylamine, n-methylbutylamine, 1-methoxy-2-propanamine, 2-methoxyethanamine, ethylpropionate, dioxane, 3-pentanone, 2-pentanone, 2-methoxy-1propanamine, n-pentylamine, 3,3-dimethyl-2-butanone, 1,3-dioxane, piperidine, 2-ethoxyethanamine, n-methyl-1,2-ethanediamine, 1,2-diaminoethane, 1,2-propanediamine, 1-(dimethylamino)-2-propanol, 2-ethylbutylamine, n-butylacetate, 2-hexanone, n-ethylethylenediamine, 1-ethoxy-2-propanol, 4-methyl-2-hexanamine, hexylamine, 2-(dimethylamino)-ethanol, cyclohexylamine, n-ethyl-2-dimethylaminoethylamine, 2-ethoxy-1-propanol, 1-methylpiperazine, 1,3-propanediamine, 2-heptanamine, n,n-diethylethylenediamine, 1-methoxy-2-acetoxypropane, 4-methylpyridine, n,n'-diethyl-1,2-ethanediamine, 2,6-dimethylmorpholine, methylhexanoate, 1-propoxy-2-propanol. Plus particulièrement, ledit agent d'extraction organique est choisi parmi le groupe consistant en éthylamine, isopropylamine, n-propylamine, diethylamine, propanone, tetrahydrofurane, ethylacetate, butanone, 3-pentylamine, 2-methoxyethanamine, dioxane, 3-pentanone, 2-pentanone, n-pentylamine, 1,3-dioxane, 1,2-diaminoethane, 1,2-propanediamine, 2-methoxyethanol, n-butylacetate, 1-ethoxy-2-propanol.

Ledit flux **G1h** comprenant 1,1,1,2,2-pentafluoropropane (245cb) peut être recyclé à l'étape a) du présent procédé. Ledit flux **G1h** comprenant 1,1,1,2,2-pentafluoropropane (245cb) peut être un desdits un ou plusieurs flux recyclé à l'étape a) du présent procédé (étape c)).

Comme expliqué ci-dessus, le courant **G1i** comprenant trans-1,3,3,3-tetrafluoro-1-propene (1234zeE) et ledit agent d'extraction organique est distillé pour séparer l'agent d'extraction organique du trans-1,3,3,3-tetrafluoro-1-propene (1234zeE), avantageusement ledit agent d'extraction organique ainsi séparé est recyclé à l'étape b4). Le trans-1,3,3,3-tetrafluoro-1-propene (1234zeE) peut être incinéré ou purifié pour être utilisé ultérieurement ou pour être vendu.

Le courant **G1e** peut être purifié, par exemple par distillation extractive, pour éliminer du trans-1,3,3,3-tetrafluoro-1-propene (1234ze-E) éventuellement présent. Dans ce cas, ledit agent d'extraction organique est un solvant choisi parmi le groupe consistant en hydrocarbure, hydrohalocarbure, alcool, cétone, amine, ester, éther, aldéhyde, nitrile, carbonate, thioalkyle, amide et hétérocycle ; ou ledit agent d'extraction organique est difluorodiethylsilane, triethylfluorosilane ou l'acide perfluorobutanoïque; de préférence parmi le groupe consistant en amine, éther, cétone, ester, alcool, aldéhyde, hétérocycle. Le point d'ébullition dudit agent d'extraction organique peut être compris entre 10 et 150°C. Ledit agent d'extraction organique peut avoir un facteur de séparation S_{1,2} supérieur ou égal à 1,1, ledit facteur de séparation étant calculé par la formule S_{1,2} = (γ_{1,S}*P1)/(γ_{2,S}*P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du trans-1,3,3,3-tetrafluoro-1-propene (1234ze-E), dans ledit agent d'extraction organique à dilution infinie, P2 représente la pression de vapeur saturante de dudit au moins un des composés consistant en trans-1,3,3,3-tetrafluoro-1-propene (1234ze-E) ; avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0. Ledit agent d'extraction organique peut avoir une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité du trans-1,3,3,3-tetrafluoro-1-propene (1234ze-E) dans ledit agent d'extraction organique à dilution infinie ; avantageusement, la capacité d'absorption C_{2,S} est supérieure ou égale à 0,40, de préférence supérieure ou égale à 0,60, plus préférentiellement supérieure ou égale à 0,80, en particulier supérieure ou égale à 1,0. Avantageusement, ledit agent d'extraction organique peut être éthylamine, isopropylamine, diethylether, ethoxy-ethene, dimethoxymethane, n-propylamine, methyl-t-butylether, diethylamine, propanone, methylacetate, isobutanal, tetrahydrofurane, isopropylformate, diisopropylether, 2-ethoxy-2-methyl-propane, ethylacetate, butanone, diethoxymethane, isopropylacetate, 3-pentylamine, 2-methoxyethanamine, tert-butylacetate, dioxane, 3-pentanone, 1,1-diethoxyethane, 2-pentanone, trimethoxymethane, n-pentylamine, 1,3-dioxane, 3,3-dimethyl-2-butanone, sec-butylacetate, 4-methyl-2-pentanone, 1,2-diaminoethane, 1-methoxy2-propanol, diethylcarbonate, n-butylacetate, 1-ethoxy-2-propanol, hexanal ; avantageusement ledit agent d'extraction organique est choisi parmi le groupe consistant en éthylamine, isopropylamine, diethylether, dimethoxymethane, n-propylamine, , diethylamine, , diisopropylether, 2-ethoxy-2-methyl-propane, butanone, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal ; de préférence ledit agent d'extraction organique est choisi parmi le groupe consistant en éthylamine, isopropylamine, diethylether, dimethoxymethane, n-propylamine, diethylamine, diisopropylether, 2-ethoxy-2-methyl-propane, diethoxymethane, isopropylacetate, 3-pentylamine, 2-methoxyethanamine, tert-butylacetate, dioxane, trimethoxymethane, n-pentylamine, 1,3-dioxane, sec-butylacetate, 1,2-diaminoethane, 1-methoxy2-propanol, n-butylacetate, 1-ethoxy-2-propanol, hexanal.

Alternativement, comme mentionné ci-dessus, le procédé comprend une étape b3'), subséquente à l'étape b2), dans laquelle le courant **G1c** obtenu à l'étape b2) comprend 2,3,3,3-tétrafluoropropène (1234yf), 1,1,1,2,2-pentafluoropropane (245cb) et trans-1,3,3,3-tetrafluoro-1-propene (1234zeE) ; et ledit courant **G1c** est distillé pour former un courant **G1e'** comprenant 2,3,3,3-tétrafluoropropène (1234yf) et 1,1,1,2,2-pentafluoropropane (245cb) et un courant **G1f'** comprenant trans-1,3,3,3-tetrafluoro-1-propene (1234zeE). Ainsi, le courant **G1c** peut être distillé par distillation extractive suivant les étapes :
b4') mise en contact dudit courant **G1c** obtenu à l'étape b2) avec un agent d'extraction organique pour former un courant **G1g',** et
b5') distillation extractive du courant **G1g'** pour former le flux **G1e'** comprenant 2,3,3,3-tetrafluoropropene (1234yf) et 1,1,1,2,2-pentafluoropropane (245cb), avantageusement en tête de colonne de distillation, et le courant **G1h'** comprenant trans-1,3,3,3-tetrafluoro-1-propene (1234zeE) et ledit agent d'extraction organique, avantageusement en bas de colonne de distillation.

Selon un mode de réalisation préféré, ledit agent d'extraction organique peut avoir un facteur de séparation S_{1,2} supérieur ou égal à 1,1, ledit facteur de séparation étant calculé par la formule S_{1,2} = (γ_{1,S}*P1)/(γ_{2,S}*P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du trans-1,3,3,3-tetrafluoro-1-propene (1234ze-E) dans ledit agent d'extraction organique à dilution infinie, P2 représente la pression de vapeur saturante du trans-1,3,3,3-tetrafluoro-1-propene (1234ze-E) ; avantageusement, le facteur de séparation est supérieur ou égal à 1,2, de préférence supérieur ou égal à 1,4, plus préférentiellement supérieur ou égal à 1,6, en particulier supérieur ou égal à 1,8. Dans ce mode de réalisation, ledit agent d'extraction organique peut également avoir un facteur de séparation S_{1,2} supérieur ou égal à 1,1, ledit facteur de séparation étant calculé par la formule S_{1,2} = (γ_{1,S}*P1)/(γ_{2,S}*P2) dans laquelle γ_{1,S} représente le coefficient d'activité du 1,1,1,2,2-pentafluoropropane (245cb) dans ledit agent d'extraction organique à dilution infinie, P1 représente la pression de vapeur saturante du 1,1,1,2,2-pentafluoropropane (245cb), γ_{2,S} représente le coefficient d'activité du trans-1,3,3,3-tetrafluoro-1-propene (1234ze-E) dans ledit agent d'extraction organique à dilution infinie, P2 représente la pression de vapeur saturante du trans-1,3,3,3-tetrafluoro-1-propene (1234ze-E) ; avantageusement, le facteur de séparation est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0. Dans ce mode de réalisation préféré, ledit agent d'extraction organique peut avoir une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité dudit au moins un des composés consistant en trans-1,3,3,3-tetrafluoro-1-propene (1234ze-E) dans ledit agent d'extraction organique à dilution infinie, de préférence γ_{2,S} représente le coefficient d'activité dudit au moins un des composés consistant en trans-1,3,3,3-tetrafluoro-1-propene (1234ze-E), dans ledit agent d'extraction organique à dilution infinie ; avantageusement, la capacité d'absorption C_{2,S} est supérieure ou égale à 0,40, de préférence supérieure ou égale à 0,60, plus préférentiellement supérieure ou égale à 0,80, en particulier supérieure ou égale à 1,0. Ainsi, dans ce mode de réalisation préféré, ledit agent d'extraction organique peut être éthylamine, isopropylamine, diethylether, ethylmethylamine, 2-amino-2-methylpropane, n-propylamine, isopropylmethylamine, 2-ethoxy-propane, methyl-t-butylether, diethylamine, propanone, methylacetate, 2-butanamine, n-methylpropylamine, isobutanal, tetrahydrofurane, 1-butylamine, ethylacetate, butanone, n-propylformate, 2,2-dimethoxypropane, 1-ethoxy-2-methylpropane, 1,2-dimethoxyethane, 3-methyl-2-butanamine, diethoxymethane, isopropylacetate, 3-pentylamine, n-methylbutylamine, 1-ethoxybutane, 1-methoxy-2-propanamine, 2-methylbutanal, 2-methoxyethanamine, tert-butylacetate, 1-methoxy-pentane, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 4-methyl-2-pentanone, 1,2-diaminoethane, butyronitrile, 1-methoxy2-propanol, 1,2-propanediamine, 2,6-dimethyl-5-heptenal, 1-(dimethylamino)-2-propanol, 1,1-diethoxypropane, 2-ethylbutylamine, diethylcarbonate, n-butylacetate, 2-hexanone, n-ethylethylenediamine, 5-hexen-2-one, 2-methylpyridine, 2-methoxy1-propanol, hexanal ; avantageusement, éthylamine, isopropylamine, diethylether, n-propylamine, diethylamine, propanone, methylacetate, butanone, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal ; de préférence, éthylamine, isopropylamine, diethylether, n-propylamine, diethylamine, diethoxymethane, isopropylacetate, 3-pentylamine, 2-methoxyethanamine, tert-butylacetate, dioxane, 1,1-diethoxyethane, trimethoxymethane, n-pentylamine, 1,3-dioxane, sec-butylacetate, 1,2-diaminoethane, 1-methoxy-2-propanol, 1,2-propanediamine, n-butylacetate, 2-methoxy-1-propanol, hexanal.

De préférence, le courant **G1e'** comprenant 2,3,3,3-tétrafluoropropène (1234yf) et 1,1,1,2,2-pentafluoropropane (245cb) peut être soumis à des étapes de purification ultérieures. Ainsi, le 2,3,3,3-tétrafluoropropène (1234yf) peut être séparé, de préférence par distillation du 1,1,1,2,2-pentafluoropropane (245cb) pour former un courant comprenant 2,3,3,3-tétrafluoropropène (1234yf) et un courant **G1i'** comprenant 1,1,1,2,2-pentafluoropropane (245cb), ledit courant **G1i'** étant recyclé à l'étape a). Le 2,3,3,3-tétrafluoropropène (1234yf) peut encore être soumis à des étapes de purification ultérieures afin d'obtenir un degré de pureté suffisant pour sa commercialisation. Par exemple, celui-ci peut être purifié, par exemple par distillation extractive, pour éliminer du trans-1,3,3,3-tetrafluoro-1-propene (1234ze-E) éventuellement présent.

De préférence, le courant **G1h'** comprenant trans-1,3,3,3-tetrafluoro-1-propene (1234zeE) et ledit agent d'extraction organique est séparé par distillation pour former un courant **G1j'** comprenant ledit agent d'extraction organique et un courant **G1k'** comprenant trans-1,3,3,3-tetrafluoro-1-propene (1234zeE). Le courant **G1j'** comprenant ledit agent d'extraction organique peut être recyclé à l'étape b4'). Le courant **G1k'** comprenant trans-1,3,3,3-tetrafluoro-1-propene (1234zeE) peut être soit purifié ou détruit par incinération.

Si le courant gazeux **G1** comprend éventuellement après distillation du HF, celui-ci peut être contenu dans ledit courant **G1b,** puis **G1c.** Ainsi, préalablement à l'étape b3), le HF n'ayant pas réagi contenu dans le courant **G1c** peut être éliminé dudit courant **G1c.** L'élimination de l'HF peut être effectuée par une série d'étape d'absorption de celui-ci en milieu aqueux. Le courant **G1c** peut ensuite être neutralisé en présence d'une base, par exemple un hydroxyde alcalin ou alcalino-terreux, puis séché, par exemple sur tamis moléculaire pour enlever d'éventuellement traces d'eau. Optionnellement ou non, si le courant **G1c** comprend des impuretés ayant un point d'ébullition inférieur à celui du 2,3,3,3-tetrafluoro-1-propène, celles-ci peuvent être éliminées par distillation. Lesdites impuretés ayant un point d'ébullition inférieur à celui du 2,3,3,3-tetrafluoro-1-propène peuvent être trifluorométhane (F23), monofluorométhane (F41), difluorométhane (F32), pentafluoroéthane (F125), 1,1,1-trifluoroéthane (F143a), trifluoropropyne ou 1-chloro-pentafluoroéthane (F115) ; et celles-ci peuvent être récupérées en tête de colonne de distillation. Le courant récupéré en bas de colonne de distillation peut ensuite être utilisé comme décrit ci-dessus pour le courant **G1c** dans l'étape b) et les étapes subséquentes.

Selon un autre mode de réalisation préféré, ledit courant liquide **L1** comprend tout ou partie des produits intermédiaires **B** et tout ou partie des produits secondaires **C** ; et tout ou partie de ce courant **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C.** Ainsi, ledit courant liquide **L1** peut comprendre une partie des produits intermédiaires **B** et une partie des produits secondaires **C** ; et tout ou partie de ce courant **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C.** Avantageusement, ladite basse température est comprise entre - 50°C et 15°C, de préférence entre -40°C et 10°C, en particulier entre-30°C et 0°C. Cette étape peut être effectuée de façon continue ou discontinue.

Ladite première phase **L1a** peut être recyclée à l'étape a).

Optionnellement ou non, ledit courant **G1d** formé à l'étape b2) peut être mélangé au courant liquide **L1** avant que tout ou partie de ce dernier soit porté à basse température.

Tout ou partie du 1,1,1,2,2-pentafluoropropane (245cb) peut aussi être contenu dans ledit courant liquide **L1** puis dans ladite seconde phase **L1b.**

Tout ou partie du 1,3,3,3-tetrafluoro-1-propene (1234ze) peut aussi être contenu dans ledit courant liquide **L1** puis dans ladite seconde phase **L1b.**

Tout ou partie du 2-chloro-3,3,3-trifluoropropène (1233xf) peut aussi être contenu dans ledit courant liquide **L1** puis dans ladite seconde phase **L1b.**

Tout ou partie du E-1-chloro-3,3,3-trifluoro-1-propene (1233zdE) peut aussi être contenu dans ledit courant liquide **L1** puis dans ladite seconde phase **L1b.**

Tout ou partie du 1,1,1,3,3-pentafluoropropane (245fa) peut aussi être contenu dans ledit courant liquide **L1** puis dans ladite seconde phase **L1b.**

De préférence, ladite seconde phase **L1b** peut comprendre 1,1,1,3,3-pentafluoropropane (245fa), E-1-chloro-3,3,3-trifluoro-1-propene (1233zdE), 2-chloro-3,3,3-trifluoropropène (1233xf), 1,3,3,3-tetrafluoro-1-propene (1234ze) et 1,1,1,2,2-pentafluoropropane (245cb).

Selon un mode de réalisation préféré, ladite seconde phase **L1b** est distillée pour récupérer un courant **L1c** comprenant 1,1,1,2,2-pentafluoropropane (245cb) et 1,3,3,3-tetrafluoro-1-propene (1234ze), avantageusement en tête de colonne de distillation, et un courant **L1d** comprenant 2-chloro-3,3,3-trifluoro-1-propene (1233xf), E-1-chloro-3,3,3-trifluoro-1-propene (1233zdE) et 1,1,1,3,3-pentafluoropropane (245fa) ; avantageusement en bas de colonne de distillation. Ledit courant **L1c** peut être recyclé à l'étape a). Ledit courant **L1c** peut donc être un desdits un ou plusieurs flux recyclé à l'étape a) lors de l'étape c) du présent procédé. Optionnellement, ledit courant **L1c** peut être purifié pour séparer 1,1,1,2,2-pentafluoropropane (245cb) et 1,3,3,3-tetrafluoro-1-propene (1234ze). Ceci peut être effectué par distillation extractive tel qu'explicité ci-dessus en relation avec la séparation du courant **G1f.**

Selon un mode de réalisation préféré, ledit courant **L1d** peut être séparé pour former un flux comprenant 2-chloro-3,3,3-trifluoro-1-propene (1233xf) et un courant comprenant E-1-chloro-3,3,3-trifluoro-1-propene (1233zdE) et 1,1,1,3,3-pentafluoropropane (245fa). La séparation dudit courant **L1d** peut être effectuée par distillation extractive.

De préférence, ledit courant **L1d** peut être une composition azéotropique ou quasi-azéotropique comprenant 2-chloro-3,3,3-trifluoro-1-propène (1233xf), E-1-chloro-3,3,3-trifluoro-1-propene (1233zdE) et 1,1,1,3,3-pentafluoropropane (245fa).

De préférence, ladite séparation peut être effectuée par distillation extractive. Ladite distillation extractive dudit courant **L1d** comprend les étapes de :
- mise en contact dudit courant **L1d** avec un agent d'extraction organique pour former une composition **L1e**, et
- distillation extractive de la composition **L1e** pour former un flux **L1f** comprenant 2-chloro-3,3,3-trifluoro-1-propene (1233xf), avantageusement en tête de colonne de distillation, et un courant **L1g** comprenant E-1-chloro-3,3,3-trifluoro-1-propene (1233zdE) et 1,1,1,3,3-pentafluoropropane (245fa) et ledit agent d'extraction organique, avantageusement en bas de colonne de distillation.

De préférence, le courant **L1g** est ensuite séparé par distillation pour former un courant **L1h** comprenant ledit agent d'extraction organique et un courant **L1i** comprenant E-1-chloro-3,3,3-trifluoro-1-propene (1233zdE) et 1,1,1,3,3-pentafluoropropane (245fa). Le courant **L1h** peut être recyclé pour être mis en contact avec un courant **L1d** pour former une composition **L1e.** Le courant **L1i** comprenant E-1-chloro-3,3,3-trifluoro-1-propene (1233zdE) et 1,1,1,3,3-pentafluoropropane (245fa) peut être soit purifié soit détruit par incinération.

Selon un mode de réalisation préféré, ledit agent d'extraction organique mis en contact avec le courant **L1d** est un solvant choisi parmi le groupe consistant en hydrocarbure, hydrohalocarbure, alcool, cétone, amine, ester, éther, aldéhyde, nitrile, carbonate, sulfoxide, sulfate, thioalkyle, amide, hétérocycle et phosphate ou l'agent d'extraction organique est l'acide perfluorobutanoïque. Selon un mode de réalisation préféré, ledit agent d'extraction organique a un point d'ébullition compris entre 50 et 200°C. Selon un mode de réalisation préféré, ledit agent d'extraction organique a un facteur de séparation S_{1,2} supérieur ou égal à 1,1, ledit facteur de séparation étant calculé par la formule S_{1,2} = (γ_{1,S}*P1)/(γ_{2,S}*P2) dans laquelle γ_{1,S} représente le coefficient d'activité du 2-chloro-3,3,3-trifluoropropène dans ledit agent d'extraction organique à dilution infinie,
P1 représente la pression de vapeur saturante du 2-chloro-3,3,3-trifluoropropène,
γ_{2,S} représente le coefficient d'activité du 1,1,1,3,3-pentafluoropropane (245fa) dans ledit agent d'extraction organique à dilution infinie,
P2 représente la pression de vapeur saturante du 1,1,1,3,3-pentafluoropropane (245fa); avantageusement, le facteur de séparation S_{1,2} est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0 ;
et
ledit agent d'extraction organique a une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité du 1,1,1,3,3-pentafluoropropane (245fa) dans ledit agent d'extraction organique à dilution infinie ; avantageusement, la capacité d'absorption C_{2,S} est supérieure ou égale à 0,40, de préférence supérieure ou égale à 0,60, plus préférentiellement supérieure ou égale à 0,80, en particulier supérieure ou égale à 1,0.

Ainsi, selon un mode de réalisation particulier, ledit agent d'extraction organique peut être choisi parmi le groupe consistant en éthanedial, propanone, methylacetate, methylglyoxal, ethylacetate, butanone, propionitrile, dioxane, trimethoxymethane, 1,3-dioxane, 1,3,5-trioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, methylacetoacetate, n,n-dimethylpropanamide, dimethylmalonate, diethylsulfoxide, 2-(2-methoxyethoxy)ethanol, trimethylphosphate, diethylmalonate ; de préférence ledit agent d'extraction organique peut être choisi parmi le groupe consistant en propanone, methylacetate, ethylacetate, butanone, dioxane, trimethoxymethane, 1,3-dioxane, 1,3,5-trioxane, 1,2-diaminoethane, 1-methoxy-2-propanol. De préférence, ce mode de réalisation particulier peut permettre de séparer efficacement 2-chloro-3,3,3-trifluoropropène et 1,1,1,3,3-pentafluoropropane (245fa).

Selon un mode de réalisation particulier, ledit agent d'extraction organique mis en contact avec le courant **L1d** peut avoir un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 2-chloro-3,3,3-trifluoropropène dans ledit agent d'extraction organique à dilution infinie,
P1 représente la pression de vapeur saturante du 2-chloro-3,3,3-trifluoropropène,
γ_{2,S} représente le coefficient d'activité du E-1-chloro-3,3,3-trifluoro-1-propene (1233zdE) dans ledit agent d'extraction organique à dilution infinie,
P2 représente la pression de vapeur saturante du E-1-chloro-3,3,3-trifluoro-1-propene (1233zdE);
avantageusement, le facteur de séparation S_{1,2} est supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0 ;
et
ledit agent d'extraction organique peut avoir une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité du E-1-chloro-3,3,3-trifluoro-1-propene (1233zdE) dans ledit agent d'extraction organique à dilution infinie ;
avantageusement, la capacité d'absorption C_{2,S} est supérieure ou égale à 0,40, de préférence supérieure ou égale à 0,60, plus préférentiellement supérieure ou égale à 0,8, en particulier supérieur ou égale à 1,0.

Ainsi, dans un mode de réalisation particulier, ledit agent d'extraction organique peut être choisi parmi le groupe consistant en isopropylmethylamine, methyl-t-butylether, diethylamine, propanone, methylacetate, 2-butanamine, n-methylpropylamine, tetrahydrofurane, 1-butylamine, ethylacetate, butanone, n-propylformate, -dimethoxypropane, diisopropylamine, 1,2-dimethoxyethane, 3-methyl-2-butanamine, diethoxymethane, isopropylacetate, 3-pentylamine, n-methylbutylamine, 1-methoxy-2-propanamine, 2-methoxyethanamine, tert-butylacetate, ethylpropionate, 1,2-dimethoxypropane, dioxane, 3-pentanone, 1,1-diethoxyethane, 2-pentanone, 2-methoxy-1propanamine, trimethoxymethane, n-pentylamine, 3,3-dimethyl-2-butanone, 1,3-dioxane, piperidine, 2-ethoxyethanamine, sec-butylacetate, n-methyl-1,2-ethanediamine, 2,2-diethoxypropane, 1,2-diaminoethane, 1-methoxy2-propanol, 1,2-propanediamine, 2,6-dimethyl-5-heptenal, 1-(dimethylamino)-2-propanol, 3-methyl-3-pentanol, 2-ethylbutylamine, diethylcarbonate, n-butylacetate, 2-hexanone, n-ethylethylenediamine, 2-methoxy1-propanol, 1-ethoxy-2-propanol, 4-methyl-2-hexanamine, hexylamine, methoxycyclohexane, 2-(dimethylamino)-ethanol, cyclohexylamine, n-ethyl-2-dimethylaminoethylamine, ethoxyethanol, 2-ethoxy-1-propanol, 1-methylpiperazine, 1,3-propanediamine, 2-heptanamine, n,n-diethylethylenediamine, 4-methyl-2-hexanone, 1,1,1-triethoxyethane, 1-methoxy-2-acetoxypropane, 4-methylpyridine, n,n'-diethyl-1,2-ethanediamine, 2,6-dimethylmorpholine, methylhexanoate, 2-propoxyethanol, 1-propoxy-2-propanol, 2-heptanone, dimethylformamide, 2-isopropoxyethanol, 2-methylpiperazine, cyclohexanone, 1-heptanamine, 2-ethoxyethanolacetate, 1,4-butanediamine, 2,4-dimethylpyridine, 2-methoxy-3-methylpyrazine, 4-methoxy-4-methyl-pentan-2-one, 3-ethoxy-1-propanol, 3-methoxy-1-butanol, diglyme, 2-(diethylamino)-ethanol, 2,2-diethoxyethanamine, 2-methoxy-n-(2-methoxyethyl)ethanamine, 2-(ethylamino)ethanol, 3-octanone, diacetone alcohol, diethylaminopropylamine, 2-ethylhexylamine, 1-butoxy-2-propanol, 2-butoxyethanol, 2-octanone, methylheptanoate, triethylenediamine, n,n-dimethylpropanamide, 2-propanol-1-methoxy-propanoate, 1,5-pentanediamine, cycloheptanone, 3,4-dimethylpyridine, 1-octanamine, benzylmethylamine, 1,1,3,3-tetramethoxypropane, dihexylphthalate, diethylpropanolamine, 2-butoxyethanolacetate, diethylsulfoxide, 2-(2-methoxyethoxy)ethanol, 4-methylbenzenemethanamine, diethyleneglycolmonoethylether, 2-propylcyclohexanone, trimethylphosphate, 2-methyl-2,4-pentanediol, methylbenzoate, diethylmalonate, 2-methoxypyrimidine ; de préférence ledit agent d'extraction organique est choisi parmi le groupe consistant en diethylamine, propanone, methylacetate, tetrahydrofurane, ethylacetate, butanone, diethoxymethane, isopropylacetate, tert-butylacetate, dioxane, 3-pentanone, 1,1-diethoxyethane, 2-pentanone, n-pentylamine, 1,3-dioxane, sec-butylacetate, 1,2-diaminoethane, 1-methoxy2-propanol, n-butylacetate, 1-ethoxy-2-propanol. De préférence, ce mode de réalisation particulier peut permettre de séparer efficacement 2-chloro-3,3,3-trifluoropropène et E-1-chloro-3,3,3-trifluoro-1-propene (1233zdE).

Selon un mode de réalisation préféré, pour favoriser l'élimination simultanée de E-1-chloro-3,3,3-trifluoro-1-propene (1233zdE) et 1,1,1,3,3-pentafluoropropane (245fa) ; ledit agent d'extraction organique mis en contact avec le courant **L1d** peut être sélectionné parmi le groupe consistant en propanone, methylacetate, ethylacetate, butanone, dioxane, trimethoxymethane, 1,3-dioxane, 1,2-diaminoethane, 1-methoxy2-propanol, diethylcarbonate, 2-methoxy1-propanol, 1-methoxy-2-acetoxypropane, dimethylformamide, 3-methoxy-1-butanol, diacetone alcohol, n,n-dimethylpropanamide, diethylsulfoxide, 2-(2-methoxyethoxy)ethanol, trimethylphosphate, diethylmalonate. En particulier, ledit agent d'extraction organique peut être sélectionné parmi le groupe consistant en propanone, methylacetate, ethylacetate, butanone, dioxane, trimethoxymethane, 1,3-dioxane, 1,2-diaminoethane, 1-methoxy2-propanol, 3-methoxy-1-butanol, diacetone alcohol.

Selon un mode de réalisation préféré, ledit courant **L1g** comprenant E-1-chloro-3,3,3-trifluoro-1-propene (1233zdE), 1,1,1,3,3-pentafluoropropane (245fa) et ledit agent d'extraction organique peut être distillé pour séparer d'une part ledit agent d'extraction organique et d'autre part le E-1-chloro-3,3,3-trifluoro-1-propene (1233zdE) et le 1,1,1,3,3-pentafluoropropane (245fa). De préférence, ledit agent d'extraction organique peut être recyclé.

Selon un mode de réalisation préféré, le flux **L1f** comprenant 2-chloro-3,3,3-trifluoro-1-propene (1233xf) est recyclé à l'étape a).

Si des impuretés lourdes sont présentes dans ledit courant **L1d**, celui-ci peut être distillé préalablement à sa séparation pour éliminer celles-ci. Le courant **L1d** tel que décrit ci-dessus peut être récupéré en tête de colonne de distillation, les impuretés lourdes étant récupérées en bas de colonne de distillation. Les impuretés lourdes peuvent contenir par exemple 1,2-dichloro-3,3,3-trifluoropropene (1223xd), des dimères ou des trimères issus de l'un des composés présents dans la composition ou le courant considéré.

Plus particulièrement, la composition de départ peut comprendre 1,1,2,3-tetrachloropropène, le 2,3,3,3,-tetrachloropropène, le 1,1,3,3-tetrachloropropène, le 1,3,3,3-tetrachloropropène, le 1,1,1,2,3-pentachloropropane, le 1,1,1,3,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoropropane, de préférence 1,1,1,2,3-pentachloropropane, 1,1,2,3,tetrachloropropène, du 1,1,1,2,2-pentafluoropropane et/ou du 2-chloro-3,3,3-trifluoro-1-propène ; en particulier 1,1,1,2,3-pentachloropropane (240db).

Le catalyseur utilisé dans le présent procédé de production de 2,3,3,3-tétrafluoropropène peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 I.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bar, de préférence de 2 à 18 bar et plus particulièrement de 3 à 15 bars;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

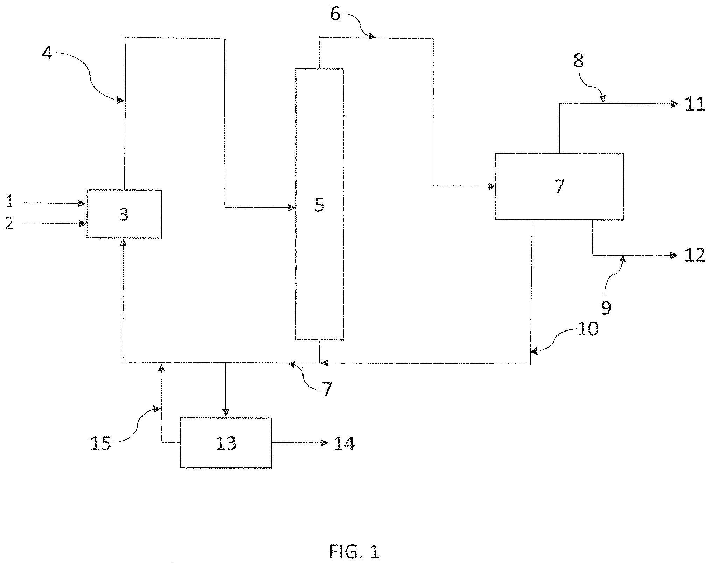

La Fig. 1 illustre schématiquement un dispositif mettant en œuvre un procédé de production du 2,3,3,3-tetrafluoropropène selon un mode de réalisation particulier de la présente invention. L'acide fluorhydrique 1 est mis en contact avec du 1,1,1,2,3-pentachloropropane (240db) 2 dans un ou plusieurs réacteurs 3. Le mélange obtenu et comprenant 2,3,3,3-tetrafluoro-1-propène, 1,1,1,2,2-pentafluoropropane (245cb), trans-1,3,3,3-tetrafluoro-1-propene (1234zeE), 2-chloro-3,3,3-trifluoropropène (1233xf), E-1-chloro-3,3,3-trifluoro-1-propene (1233zdE) et 1,1,1,3,3-pentafluoropropane (245fa) est récupéré en sortie de réacteur et acheminé vers une colonne de distillation 5 par le conduit 4. Le mélange peut aussi comprendre HCl, HF n'ayant pas réagi et des impuretés lourdes ou des impuretés ayant un point d'ébullition inférieur à celui du 2,3,3,3-tetrafluoro-1-propène. Tout ou partie du courant obtenu en bas de colonne de distillation est acheminé vers le dispositif de purification 13 via le conduit 7. De ce dispositif de purification 13 peuvent être extraits HF, 2-chloro-3,3,3-trifluoropropène (1233xf) et 1,1,1,2,2-pentafluoropropane (245cb), et optionnellement trans-1,3,3,3-tetrafluoro-1-propene (1234zeE), qui sont recyclés dans le réacteur 3 via le conduit 15. E-1-chloro-3,3,3-trifluoro-1-propene (1233zdE) et 1,1,1,3,3-pentafluoropropane (245fa) peuvent également être extraits du dispositif 13 pour être évacués en 14 vers un incinérateur ou un dispositif de purification. Un courant est également récupéré en tête de la colonne de distillation 5 et acheminé vers un dispositif de purification 7 via le conduit 6. Du dispositif de purification 7, un courant comprenant 2,3,3,3-tetrafluoro-1-propène est récupéré en 11 via le conduit 8. Un flux comprenant 1,1,1,2,2-pentafluoropropane (245cb), et optionnellement trans-1,3,3,3-tetrafluoro-1-propene (1234zeE), est obtenu également et recyclé vers le réacteur 3 par le conduit 10. Enfin, un courant comprenant trans-1,3,3,3-tetrafluoro-1-propene (1234zeE) peut être récupéré en 12 par l'intermédiaire du conduit 9.

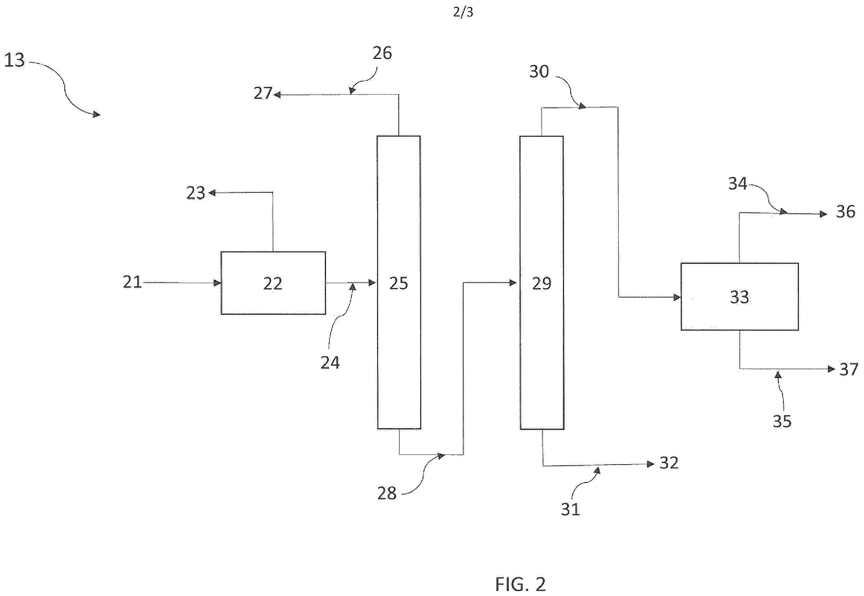

La Fig. 2 illustre schématiquement selon un mode de réalisation particulier de la présente invention un dispositif de purification 13. Un courant 21 comprenant notamment HF, trans-1,3,3,3-tetrafluoro-1-propene (1234zeE), 1,1,1,2,2-pentafluoropropane (245cb), 2-chloro-3,3,3-trifluoropropène (1233xf), E-1-chloro-3,3,3-trifluoro-1-propene (1233zdE) et 1,1,1,3,3-pentafluoropropane (245fa) est acheminé vers le décanteur 22 ayant une température de -25°C. Le HF est extrait et récupéré en 23 pour être recyclé vers le réacteur 3. Les autres constituants sont acheminés vers la colonne de distillation 25 via le conduit 24. Le trans-1,3,3,3-tetrafluoro-1-propene (1234zeE) et 1,1,1,2,2-pentafluoropropane (245cb) sont évacués en tête de colonne de distillation et récupérés en 27 via le conduit 26. Ceux-ci peuvent être récupérés pour être recyclé vers le réacteur 3. Le 2-chloro-3,3,3-trifluoropropène (1233xf), E-1-chloro-3,3,3-trifluoro-1-propene (1233zdE) et 1,1,1,3,3-pentafluoropropane (245fa) sont acheminés vers la colonne de distillation 29 via le conduit 28 pour extraire en bas de colonne de distillation des impuretés lourdes éventuellement présentes et les acheminer vers un incinérateur 32 via le conduit 31. Le 2-chloro-3,3,3-trifluoropropène (1233xf), E-1-chloro-3,3,3-trifluoro-1-propene (1233zdE) et 1,1,1,3,3-pentafluoropropane (245fa) récupérés en tête de la colonne de distillation 29 sont acheminés vers un dispositif de purification 33 via le conduit 30. De ce dispositif de purification 33, le 2-chloro-3,3,3-trifluoropropène (1233xf) peut être extrait en 36 via le conduit 34. Le dispositif de purification 33 peut être une distillation extractive. Le E-1-chloro-3,3,3-trifluoro-1-propene (1233zdE) et 1,1,1,3,3-pentafluoropropane (245fa) peuvent être récupérés en 37 via le conduit 35 pour être incinérés ou purifiés.

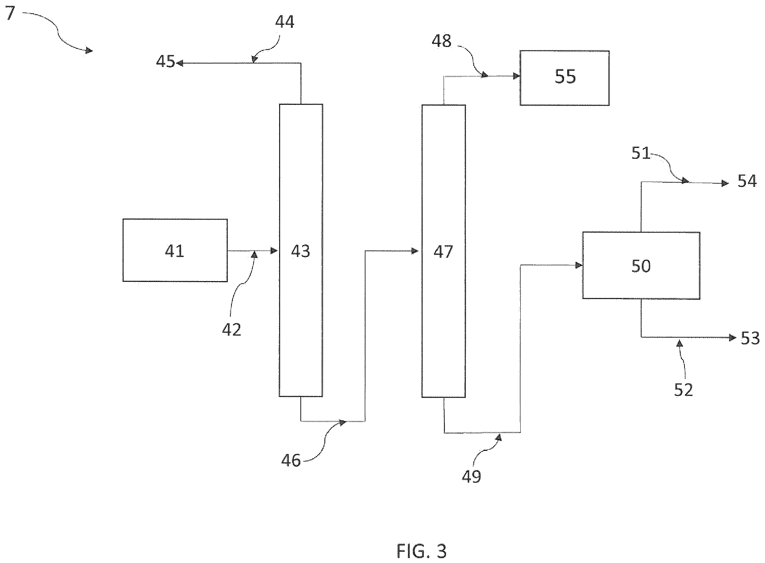

La Fig. 3 illustre schématiquement selon un mode de réalisation particulier de la présente invention un dispositif de purification 7. Une cuve de stockage 41 comprend 2,3,3,3-tetrafluoro-1-propène, trans-1,3,3,3-tetrafluoro-1-propene (1234zeE), 1,1,1,2,2-pentafluoropropane (245cb) et des impuretés ayant un point d'ébullition inférieur à celui du 2,3,3,3-tetrafluoro-1-propène. Ce mélange est acheminé vers la colonne de distillation 43 via le conduit 42. Les impuretés ayant un point d'ébullition inférieur à celui du 2,3,3,3-tetrafluoro-1-propène sont évacuées en 45 via le conduit 44. Les autres constituants du mélange sont acheminés vers la colonne de distillation 47 via le conduit 46. Un courant comprenant du 2,3,3,3-tetrafluoro-1-propène est récupéré en tête de colonne de distillation et évacué en 48 vers des cuves de stockage ou un dispositif supplémentaire de purification 55, par exemple un dispositif de distillation extractive. Le trans-1,3,3,3-tetrafluoro-1-propene (1234zeE), 1,1,1,2,2-pentafluoropropane (245cb) sont récupérés en bas de colonne de distillation pour être acheminés vers un dispositif de purification 50 via le conduit 49. Un courant comprenant 1,1,1,2,2-pentafluoropropane (245cb) séparé du trans-1,3,3,3-tetrafluoro-1-propene (1234zeE) est récupéré en 53 par le conduit 52. Le courant comprenant 1,1,1,2,2-pentafluoropropane (245cb) récupéré en 53 peut être recyclé vers le réacteur 3. Un courant comprenant trans-1,3,3,3-tetrafluoro-1-propene (1234zeE) est récupéré en 54 via le conduit 51 pour être incinéré ou purifié. Le dispositif de purification 50 peut être une distillation extractive.

## Revendications

1. Procédé de production et de purification du 2,3,3,3-tétrafluoropropène (1234yf) mis en œuvre à partir d'une composition de départ comprenant au moins un composé de formule (I) CX(Y)₂-CX(Y)ₘ-CHₘXY dans laquelle X et Y représentent indépendamment un atome d'hydrogène, de fluor ou de chlore et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ; ledit procédé comprenant les étapes de :
a) mise en contact, en présence d'un catalyseur, de la composition de départ avec HF pour produire une composition **A** comprenant HCl, une partie du HF n'ayant pas réagi, 2,3,3,3-tétrafluoropropène (1234yf), des produits intermédiaires **B** consistant en 2-chloro-3,3,3-trifluoropropène (1233xf), 1,1,1,2,2-pentafluoropropane (245cb), et des produits secondaires **C** consistant en E-1-chloro-3,3,3-trifluoro-1-propene (1233zdE), trans-1,3,3,3-tetrafluoro-1-propene (1234zeE) et 1,1,1,3,3-pentafluoropropane (245fa);
b) récupération de ladite composition **A** et purification, de préférence distillation, de celle-ci pour former et récupérer un premier courant, de préférence gazeux, comprenant HCl, 2,3,3,3-tétrafluoropropène (1234yf), une partie des produits intermédiaires **B** et une partie des produits secondaires **C** ; et un courant, de préférence liquide, **L1** comprenant une partie du HF n'ayant pas réagi, une partie des produits intermédiaires **B** et une partie des produits secondaires **C** ;
c) purification dudit premier courant pour former un courant comprenant une portion de ladite partie des produits intermédiaires **B** et une portion de ladite partie des produits secondaires **C** et recyclage de celui-ci à l'étape a).

2. Procédé selon l'une quelconque des revendications précédentes **caractérisé en ce que** ledit premier courant est un courant gazeux **G1** purifié par les étapes suivantes :
b1) distillation du courant gazeux **G1** pour récupérer un courant **G1a** comprenant du HCl, avantageusement en tête de colonne de distillation, et un courant **G1b** comprenant 2,3,3,3-tétrafluoropropène (1234yf), ladite une partie des produits intermédiaires **B** et ladite une partie des produits secondaires **C,** avantageusement en bas de colonne de distillation ;
b2) distillation dudit courant **G1b** obtenu à l'étape b1) pour former un courant **G1c** comprenant 2,3,3,3-tétrafluoropropène (1234yf), une portion de ladite partie des produits intermédiaires **B** et une portion de ladite partie des produits secondaires **C,** avantageusement en tête de colonne de distillation, et un courant **G1d** comprenant une portion de ladite partie des produits intermédiaires **B** et une portion de ladite partie des produits secondaires **C,** avantageusement en bas de colonne de distillation, de préférence le courant **G1d** est recyclé à l'étape a).

3. Procédé selon la revendication 2 **caractérisé en ce que** le procédé comprend une étape b3), subséquente à l'étape b2), dans laquelle le courant **G1c** obtenu à l'étape b2) comprend 2,3,3,3-tétrafluoropropène (1234yf), 1,1,1,2,2-pentafluoropropane (245cb) et trans-1,3,3,3-tetrafluoro-1-propene (1234zeE) ; et ledit courant **G1c** est distillé pour former un courant **G1e** comprenant 2,3,3,3-tétrafluoropropène (1234yf) et un courant **G1f** comprenant 1,1,1,2,2-pentafluoropropane (245cb) et trans-1,3,3,3-tetrafluoro-1-propene (1234zeE).

4. Procédé selon la revendication précédente **caractérisé en ce que** le courant **G1f** obtenu à l'étape b3) est séparé par distillation extractive.

5. Procédé selon la revendication précédente **caractérisé en ce que** le courant **G1f** obtenu à l'étape b3) est séparé par distillation extractive suivant les étapes :
b4) mise en contact dudit courant **G1f** obtenu à l'étape b3) avec un agent d'extraction organique pour former un courant **G1g,** et
b5) distillation extractive du courant **G1g** pour former un flux **G1h** comprenant 1,1,1,2,2-pentafluoropropane (245cb), avantageusement en tête de colonne de distillation, et un courant **G1i** comprenant trans-1,3,3,3-tetrafluoro-1-propene (1234zeE) et ledit agent d'extraction organique, avantageusement en bas de colonne de distillation.

6. Procédé selon la revendication précédente **caractérisé en ce que** le flux **G1h** comprenant 1,1,1,2,2-pentafluoropropane (245cb), de préférence dépourvu de trans-1,3,3,3-tetrafluoro-1-propene (1234zeE), est recyclé à l'étape a).

7. Procédé selon l'une quelconque des revendications 1 ou 2 **caractérisé en ce que** le procédé comprend une étape b3'), subséquente à l'étape b2), dans laquelle le courant **G1c** obtenu à l'étape b2) comprend 2,3,3,3-tétrafluoropropène (1234yf), 1,1,1,2,2-pentafluoropropane (245cb) et trans-1,3,3,3-tetrafluoro-1-propene (1234zeE) ; et ledit courant **G1c** est distillé pour former un courant **G1e'** comprenant 2,3,3,3-tétrafluoropropène (1234yf) et 1,1,1,2,2-pentafluoropropane (245cb) et un courant **G1f'** comprenant trans-1,3,3,3-tetrafluoro-1-propene **(1234zeE).**

8. Procédé selon la revendication précédente **caractérisé en ce que** le courant **G1c** est distillé par distillation extractive suivant les étapes :
b4') mise en contact dudit courant **G1c** obtenu à l'étape b2) avec un agent d'extraction organique pour former un courant **G1g',** et
b5') distillation extractive du courant **G1g'** pour former le flux **G1e'** comprenant 2,3,3,3-tetrafluoropropene (1234yf) et 1,1,1,2,2-pentafluoropropane (245cb), avantageusement en tête de colonne de distillation, et le courant **G1h'** comprenant trans-1,3,3,3-tetrafluoro-1-propene (1234zeE) et ledit agent d'extraction organique, avantageusement en bas de colonne de distillation.

9. Procédé selon l'une quelconque des revendications précédentes **caractérisé en ce que** ledit courant liquide **L1** comprend une partie des produits intermédiaires **B** et tout ou partie des produits secondaires C, et une partie du courant liquide **L1** est porté à basse température, avantageusement entre -50°C et 20°C, pour former une première phase **L1a** comprenant une partie du HF n'ayant pas réagi et une seconde phase **L1b** comprenant lesdits produits intermédiaires **B** et lesdits produits secondaires **C** ; optionnellement ou non, ledit courant **G1d** formé à l'étape b2) est mélangé au courant liquide **L1** avant que ce dernier soit porté à basse température.

10. Procédé selon la revendication précédente **caractérisé en ce que** ladite première phase **L1a** est recyclée à l'étape a).

11. Procédé selon la revendication 9 ou 10 **caractérisé en ce que** ladite seconde phase **L1b** est distillée pour récupérer un courant **L1c** comprenant 1,1,1,2,2-pentafluoropropane (245cb) et trans-1,3,3,3-tetrafluoro-1-propene (1234zeE), avantageusement en tête de colonne de distillation, et un courant **L1d** comprenant 2-chloro-3,3,3-trifluoro-1-propene (1233xf), E-1-chloro-3,3,3-trifluoro-1-propene (1233zdE) et 1,1,1,3,3-pentafluoropropane (245fa) ; avantageusement en bas de colonne de distillation, avantageusement ledit courant **L1c** est recyclé à l'étape a).

12. Procédé selon la revendication précédente **caractérisé en ce que** ledit courant **L1d** est séparé pour former un flux comprenant 2-chloro-3,3,3-trifluoro-1-propene (1233xf) et un courant comprenant E-1-chloro-3,3,3-trifluoro-1-propene (1233zdE) et 1,1,1,3,3-pentafluoropropane (245fa).

13. Procédé selon la revendication précédente **caractérisé en ce que** la séparation dudit courant **L1d** est effectuée par distillation extractive.

14. Procédé selon la revendication précédente **caractérisé en ce que** la distillation extractive dudit courant **L1d** comprend les étapes de :
- mise en contact dudit courant **L1d** avec un agent d'extraction organique pour former une composition **L1e**, et
- distillation extractive de la composition **L1e** pour former un flux **L1f** comprenant 2-chloro-3,3,3-trifluoro-1-propene (1233xf), avantageusement en tête de colonne de distillation, et un courant **L1g** comprenant E-1-chloro-3,3,3-trifluoro-1-propene (1233zdE) et 1,1,1,3,3-pentafluoropropane (245fa) et ledit agent d'extraction organique, avantageusement en bas de colonne de distillation.

15. Procédé selon la revendication précédente **caractérisé en ce que** le flux **L1f** comprenant 2-chloro-3,3,3-trifluoro-1-propene (1233xf), de préférence dépourvu de E-1-chloro-3,3,3-trifluoro-1-propene (1233zdE) et/ou de 1,1,1,3,3-pentafluoropropane (245fa) est recyclé à l'étape a).

## Patentansprüche

1. Verfahren zur Herstellung und Reinigung von 2,3,3,3-Tetrafluorpropen (1234yf), durchgeführt ausgehend von einer Ausgangszusammensetzung, die mindestens eine Verbindung der Formel (I) CX(Y)₂-CX(Y)ₘ-CHₘXY, worin X und Y unabhängig für ein Wasserstoff-, Fluor- oder Chloratom stehen und m = 0 oder 1, umfasst; und/oder Fluorierung einer Verbindung der Formel (CXₙY₃₋ₙ) CHₚX₁₋ₚCHₘX₂₋ₘ (II), worin X unabhängig voneinander für Cl, F, I oder Br steht; Y unabhängig voneinander für H, Cl, F, I oder Br steht; n für 1, 2 oder 3 steht; und m für 0, 1 oder 2 steht; und p für 0 oder 1 steht; in Gegenwart eines Katalysators; wobei das Verfahren folgende Schritte umfasst:
a) Inkontaktbringen der Ausgangszusammensetzung in Gegenwart eines Katalysators mit HF zur Herstellung einer Zusammensetzung **A,** die HCl, einen Teil des nicht umgesetzten HF, 2,3,3,3-Tetrafluorpropen (1234yf), Zwischenprodukte **B,** die aus 2-Chlor-3,3,3-trifluorpropen (1233xf), 1,1,1,2,2-Pentafluorpropan (245cb) bestehen, und Nebenprodukte **C,** die aus E-1-Chlor-3,3,3-trifluor-1-propen (1233zdE), trans-1,3,3,3-Tetrafluor-1-propen (1234zeE) und 1,1,1,3,3-Pentafluorpropan (245fa) bestehen, umfasst;
b) Gewinnung der Zusammensetzung **A** und Reinigung, vorzugsweise Destillation, davon zur Bildung und Gewinnung eines ersten, vorzugsweise gasförmigen Stroms, der HCl, 2,3,3,3-Tetrafluorpropen (1234yf), einen Teil der Zwischenprodukte **B** und einen Teil der Nebenprodukte **C** umfasst; und eines vorzugsweise flüssigen Stroms **L1,** der einen Teil des nicht umgesetzten HF, einen Teil der Zwischenprodukte **B** und einen Teil der Nebenprodukte **C** umfasst;
c) Reinigung des ersten Stroms zur Bildung eines Stroms, der einen Anteil des Teils der Zwischenprodukte **B** und einen Anteil des Teils der Nebenprodukte **C** umfasst, und Zurückführung davon zum Schritt a).

2. Verfahren nach einem der vorhergehenden Ansprüche, **dadurch gekennzeichnet, dass** es sich bei dem ersten Strom um einen gasförmigen Strom **G1** handelt, der durch folgende Schritte gereinigt wird:
b1) Destillation des gasförmigen Stroms **G1** zur Gewinnung eines Stroms **G1a,** der HCl umfasst, vorteilhafterweise am Kopf der Destillationssäule, und eines Stroms **G1b,** der 2,3,3,3-Tetrafluorpropen (1234yf), den einen Teil der Zwischenprodukte **B** und den einen Teil der Nebenprodukte **C** umfasst, vorteilhafterweise am Sumpf der Destillationssäule;
b2) Destillation des in Schritt b1) erhaltenen Stroms **G1b** zur Bildung eines Stroms **G1c,** der 2,3,3,3-Tetrafluorpropen (1234yf), einen Anteil des Teils der Zwischenprodukte **B** und einen Anteil des Teils der Nebenprodukte **C** umfasst, vorteilhafterweise am Kopf der Destillationssäule, und eines Stroms **G1d,** der einen Anteil des Teils der Zwischenprodukte **B** und einen Anteil des Teils der Nebenprodukte **C** umfasst, vorteilhafterweise am Sumpf der Destillationssäule, wobei der Strom **G1d** vorzugsweise in Schritt a) zurückgeführt wird.

3. Verfahren nach einem der Ansprüche 2, **dadurch gekennzeichnet, dass** das Verfahren einen auf Schritt b2) folgenden Schritt b3) umfasst, in dem der in Schritt b2) erhaltene Strom **G1c** 2,3,3,3-Tetrafluorpropen (1234yf), 1,1,1,2,2-Pentafluorpropan (245cb) und trans-1,3,3,3-Tetrafluor-1-propen (1234zeE) umfasst und der Strom **G1c** destilliert wird, um einen Strom **Gle,** der 2,3,3,3-Tetrafluorpropen (1234yf) umfasst, und einen Strom **G1f**, der 1,1,1,2,2-Pentafluorpropan (245cb) und trans-1,3,3,3-Tetrafluor-1-propen (1234zeE) umfasst, zu bilden.

4. Verfahren nach dem vorhergehenden Anspruch, **dadurch gekennzeichnet, dass** der in Schritt b3) erhaltene Strom **G1f** durch Extraktivdestillation getrennt wird.

5. Verfahren nach dem vorhergehenden Anspruch, **dadurch gekennzeichnet, dass** der in Schritt b3) erhaltene Strom **G1f** durch Extraktivdestillation gemäß den folgenden Schritten getrennt wird:
b4) Inkontaktbringen des in Schritt b3) erhaltenen Stroms **G1f** mit einem organischen Extraktionsmittel zur Bildung eines Stroms **G1g** und
b5) Extraktivdestillation des Stroms **G1g** zur Bildung eines Stroms **G1h,** der 1,1,1,2,2-Pentafluorpropan (245cb) umfasst, vorteilhafterweise am Kopf der Destillationssäule, und eines Stroms **G1i,** der trans-1,3,3,3-Tetrafluor-1-propen (1234zeE) und das organische Extraktionsmittel umfasst, vorteilhafterweise am Sumpf der Destillationssäule.

6. Verfahren nach dem vorhergehenden Anspruch, **dadurch gekennzeichnet, dass** der Strom **G1h,** der 1,1,1,2,2-Pentafluorpropan (245cb) umfasst und vorzugsweise frei von trans-1,3,3,3-Tetrafluor-1-propen (1234zeE) ist, in Schritt a) zurückgeführt wird.

7. Verfahren nach Anspruch 1 oder 2, **dadurch gekennzeichnet, dass** das Verfahren einen auf Schritt b2) folgenden Schritt b3') umfasst, in dem der in Schritt b2) erhaltene Strom **G1c** 2,3,3,3-Tetrafluorpropen (1234yf), 1,1,1,2,2-Pentafluorpropan (245cb) und trans-1,3,3,3-Tetrafluor-1-propen (1234zeE) umfasst und der Strom **G1c** destilliert wird, um einen Strom **Gle',** der 2,3,3,3-Tetrafluorpropen (1234yf) und 1,1,1,2,2-Pentafluorpropan (245cb) umfasst, und einen Strom **G1f'**, der trans-1,3,3,3-Tetrafluor-1-propen (1234zeE) umfasst, zu bilden.

8. Verfahren nach dem vorhergehenden Anspruch, **dadurch gekennzeichnet, dass** der Strom **G1c** gemäß den folgenden Schritten durch Extraktivdestillation getrennt wird:
b4') Inkontaktbringen des in Schritt b2) erhaltenen Stroms **G1c** mit einem organischen Extraktionsmittel zur Bildung eines Stroms **G1g'** und
b5') Extraktivdestillation des Stroms **G1g'** zur Bildung des Stroms **G1e',** der 2,3,3,3-Tetrafluorpropen (1234yf) und 1,1,1,2,2-Pentafluorpropan (245cb) umfasst, vorteilhafterweise am Kopf der Destillationssäule, und des Stroms **G1h',** der trans-1,3,3,3-Tetrafluor-1-propen (1234zeE) und das organische Extraktionsmittel umfasst, vorteilhafterweise am Sumpf der Destillationssäule.

9. Verfahren nach einem der vorhergehenden Ansprüche, **dadurch gekennzeichnet, dass** der flüssige Strom **L1** einen Teil der Zwischenprodukte **B** und alle oder einen Teil der Nebenprodukte **C** umfasst und ein Teil des flüssigen Stroms **L1** auf niedrige Temperatur, vorteilhafterweise zwischen -50 °C und 20 °C, gebracht wird, um eine erste Phase **L1a,** die einen Teil des nicht umgesetzten HF umfasst, und eine zweite Phase **L1b,** die die Zwischenprodukte **B** und die Nebenprodukte **C** umfasst, zu bilden, wobei der in Schritt **b2)** gebildete Strom **G1d** gegebenenfalls mit dem flüssigen Strom **L1** gemischt wird, bevor letzterer auf niedrige Temperatur gebracht wird, oder nicht.

10. Verfahren nach dem vorhergehenden Anspruch, **dadurch gekennzeichnet, dass** die erste Phase **L1a** in Schritt a) zurückgeführt wird.

11. Verfahren nach Anspruch 9 oder 10, **dadurch gekennzeichnet, dass** die zweite Phase **L1b** destilliert wird, um einen Strom **L1c,** der 1,1,1,2,2-Pentafluorpropan (245cb) und trans-1,3,3,3-Tetrafluor-1-propen (1234zeE) umfasst, vorteilhafterweise am Kopf der Destillationssäule, und einen Strom **L1d**, der 2-Chlor-3,3,3-trifluor-1-propen (1233xf), E-1-Chlor-3,3,3-trifluor-1-propen (1233zdE) und 1,1,1,3,3-Pentafluorpropan (245fa) umfasst, vorteilhafterweise am Sumpf der Destillationssäule, zu gewinnen, wobei der Strom **L1c** vorteilhafterweise in Schritt a) zurückgeführt wird.

12. Verfahren nach dem vorhergehenden Anspruch, **dadurch gekennzeichnet, dass** der Strom **L1d** getrennt wird, um einen Strom, der 2-Chlor-3,3,3-trifluor-1-propen (1233xf) umfasst, und einen Strom, der E-1-Chlor-3,3,3-trifluor-1-propen (1233zdE) und 1,1,1,3,3-Pentafluorpropan (245fa) umfasst, zu bilden.

13. Verfahren nach dem vorhergehenden Anspruch, **dadurch gekennzeichnet, dass** die Trennung des Stroms **L1d** durch Extraktivdestillation durchgeführt wird.

14. Verfahren nach dem vorhergehenden Anspruch, **dadurch gekennzeichnet, dass** die Extraktivdestillation des Stroms **L1d** folgende Schritte umfasst:
- Inkontaktbringen des Stroms **L1d** mit einem organischen Extraktionsmittel zur Bildung einer Zusammensetzung **L1e** und
- Extraktivdestillation der Zusammensetzung **L1e** zur Bildung eines Stroms **L1f,** der 2-Chlor-3,3,3-trifluor-1-propen (1233xf) umfasst, vorteilhafterweise am Kopf der Destillationssäule, und eines Stroms **L1g,** der E-1-Chlor-3,3,3-trifluor-1-propen (1233zdE) und 1,1,1,3,3-Pentafluorpropan (245fa) und das organische Extraktionsmittel umfasst, vorteilhafterweise am Sumpf der Destillationssäule.

15. Verfahren nach dem vorhergehenden Anspruch, **dadurch gekennzeichnet, dass** der Strom **L1f,** der 2-Chlor-3,3,3-trifluor-1-propen (1233xf) umfasst und vorzugsweise frei von E-1-Chlor-3,3,3-trifluor-1-propen (1233zdE) und/oder 1,1,1,3,3-Pentafluorpropan (245fa) ist, in Schritt a) zurückgeführt wird.

## Claims

1. Process for producing and purifying 2,3,3,3-tetrafluoropropene (1234yf) which is performed using a starting composition comprising at least one compound of formula CX(Y)₂-CX(Y)ₘ-CHₘXY (I) in which X and Y independently represent a hydrogen, fluorine or chlorine atom and m = 0 or 1; and/or fluorination, in the presence of a catalyst, of a compound of formula (CXₙY₃₋ₙ)CHₚX₁₋ₚCHₘX₂₋ₘ (II) in which X is, independently of each other, Cl, F, I or Br; Y is, independently of each other, H, Cl, F, I or Br; n is 1, 2 or 3; and m is 0, 1 or 2; and p is 0 or 1; said process comprising the steps of:
a) placing the starting composition in contact, in the presence of a catalyst, with HF to produce a composition **A** comprising HCl, part of the unreacted HF, 2,3,3,3-tetrafluoropropene (1234yf), intermediate products **B** consisting of 2-chloro-3,3,3-trifluoropropene (1233xf), 1,1,1,2,2-pentafluoropropane (245cb), and side products **C** consisting of E-1-chloro-3,3,3-trifluoro-1-propene (1233zdE), trans-1,3,3,3-tetrafluoro-1-propene (1234zeE) and 1,1,1,3,3-pentafluoropropane (245fa);
b) recovery of said composition **A** and purification, preferably distillation, thereof to form and recover a first stream, which is preferably gaseous, comprising HCl, 2,3,3,3-tetrafluoropropene (1234yf), part of the intermediate products **B** and part of the side products **C;** and a stream, which is preferably liquid, **L1** comprising part of the unreacted HF, part of the intermediate products B and part of the side products **C;**
c) purification of said first stream to form a stream comprising a portion of said part of the intermediate products **B** and a portion of said part of the side products **C** and recycling thereof into step a).

2. Process according to any one of the preceding claims, **characterized in that** said first stream is a gaseous stream **G1** which is purified via the following steps:
b1) distillation of the gaseous stream **G1** to recover a stream **G1a** comprising HCl, advantageously at the top of the distillation column, and a stream **G1b** comprising 2,3,3,3-tetrafluoropropene (1234yf), said one part of the intermediate products **B** and said one part of the side products **C,** advantageously at the bottom of the distillation column;
b2) distillation of said stream **G1b** obtained in step b1) to form a stream **G1c** comprising 2,3,3,3-tetrafluoropropene (1234yf), a portion of said part of the intermediate products **B** and a portion of said part of the side products **C,** advantageously at the top of the distillation column, and a stream **G1d** comprising a portion of said part of the intermediate products **B** and a portion of said part of the side products **C,** advantageously at the bottom of the distillation column; preferably, the stream **G1d** is recycled into step a).

3. Process according to claim 2, **characterized in that** the process comprises a step b3), subsequent to step b2), in which the stream **G1c** obtained in step b2) comprises 2,3,3,3-tetrafluoropropene (1234yf), 1,1,1,2,2-pentafluoropropane (245cb) and trans-1,3,3,3-tetrafluoro-1-propene (1234zeE); and said stream **G1c** is distilled to form a stream **G1e** comprising 2,3,3,3-tetrafluoropropene (1234yf) and a stream **G1f** comprising 1,1,1,2,2-pentafluoropropane (245cb) and trans-1,3,3,3-tetrafluoro-1-propene (1234zeE).

4. Process according to the preceding claim, **characterized in that** the stream **G1f** obtained in step b3) is separated out by extractive distillation.

5. Process according to the preceding claim, **characterized in that** the stream **G1f** obtained in step b3) is separated out by extractive distillation according to the following steps:
b4) placing said stream **G1f** obtained in step b3) in contact with an organic extracting agent to form a stream **G1g**, and
b5) extractive distillation of the stream **G1g** to form a stream **G1h** comprising 1,1,1,2,2-pentafluoropropane (245cb), advantageously at the top of the distillation column, and a stream **G1i** comprising trans-1,3,3,3-tetrafluoro-1-propene (1234zeE) and said organic extracting agent, advantageously at the bottom of the distillation column.

6. Process according to the preceding claim, **characterized in that** the stream **G1h** comprising 1,1,1,2,2-pentafluoropropane (245cb), preferably freed of trans-1,3,3,3-tetrafluoro-1-propene (1234zeE), is recycled into step a).

7. Process according to either of Claims 1 and 2, **characterized in that** the process comprises a step b3'), subsequent to step b2), in which the stream **G1c** obtained in step b2) comprises 2,3,3,3-tetrafluoropropene (1234yf), 1,1,1,2,2-pentafluoropropane (245cb) and trans-1,3,3,3-tetrafluoro-1-propene (1234zeE); and said stream **G1c** is distilled to form a stream **G1e'** comprising 2,3,3,3-tetrafluoropropene (1234yf) and 1,1,1,2,2-pentafluoropropane (245cb) and a stream **G1f'** comprising trans-1,3,3,3-tetrafluoro-1-propene (1234zeE).

8. Process according to the preceding claim, **characterized in that** the stream **G1c** is distilled by extractive distillation according to the following steps:
b4') placing said stream **G1c** obtained in step b2) in contact with an organic extracting agent to form a stream **G1g'**, and
b5') extractive distillation of the stream **G1g'** to form the stream **G1e'** comprising 2,3,3,3-tetrafluoropropene (1234yf) and 1,1,1,2,2-pentafluoropropane (245cb), advantageously at the top of the distillation column, and the stream **G1h'** comprising trans-1,3,3,3-tetrafluoro-1-propene (1234zeE) and said organic extracting agent, advantageously at the bottom of the distillation column.

9. Process according to any one of the preceding claims, **characterized in that** said liquid stream **L1** comprises part of the intermediate products **B** and all or part of the side products **C,** and part of the liquid stream **L1** is brought to low temperature, advantageously between -50°C and 20°C, to form a first phase **L1a** comprising part of the unreacted HF and a second phase **L1b** comprising said intermediate products **B** and said side products **C;** optionally or not, said stream **G1d** formed in step b2) is mixed with the liquid stream **L1** before said stream is brought to low temperature.

10. Process according to the preceding claim, **characterized in that** said first phase **L1a** is recycled into step a).

11. Process according to Claim 9 or 10, **characterized in that** said second phase **L1b** is distilled to recover a stream **L1c** comprising 1,1,1,2,2-pentafluoropropane (245cb) and trans-1,3,3,3-tetrafluoro-1-propene (1234zeE), advantageously at the top of the distillation column, and a stream **L1d** comprising 2-chloro-3,3,3-trifluoro-1-propene (1233xf), E-1-chloro-3,3,3-trifluoro-1-propene (1233zdE) and 1,1,1,3,3-pentafluoropropane (245fa), advantageously at the bottom of the distillation column, advantageously, said stream **L1c** is recycled into step a).

12. Process according to the preceding claim, **characterized in that** said stream **L1d** is separated out to form a stream comprising 2-chloro-3,3,3-trifluoro-1-propene (1233xf) and a stream comprising E-1-chloro-3,3,3-trifluoro-1-propene (1233zdE) and 1,1,1,3,3-pentafluoropropane (245fa).

13. Process according to the preceding claim, **characterized in that** the separation of said stream **L1d** is performed by extractive distillation.

14. Process according to the preceding claim, **characterized in that** the extractive distillation of said stream **L1d** comprises the steps of:
- placing said stream **L1d** in contact with an organic extracting agent to form a composition **L1e**, and
- extractive distillation of composition **L1e** to form a stream **L1f** comprising 2-chloro-3,3,3-trifluoro-1-propene (1233xf), advantageously at the top of the distillation column, and a stream **L1g** comprising E-1-chloro-3,3,3-trifluoro-1-propene (1233zdE) and 1,1,1,3,3-pentafluoropropane (245fa) and said organic extracting agent, advantageously at the bottom of the distillation column.

15. Process according to the preceding claim, **characterized in that** the stream **L1f** comprising 2-chloro-3,3,3-trifluoro-1-propene (1233xf), preferably free of E-1-chloro-3,3,3-trifluoro-1-propene (1233zdE) and/or 1,1,1,3,3-pentafluoropropane (245fa), is recycled into step a).
